# FASCICULE DE BREVET EUROPEEN

(11) **EP 3 468 369 B1**
(45) Date de publication et mention de la délivrance du brevet: **25.02.2026**
(21) Numéro de dépôt: 17728566.5
(22) Date de dépôt: 08.06.2017
(51) Int. Cl.: A01N 65/03, A01P 1/00, A01P 3/00

(54) **UTILISATION D'UN EXTRAIT CELLULAIRE D'UNE OU PLUSIEURS MICRO-ALGUES DU GENRE AMPHIDINIUM POUR SON ACTIVITÉ FONGICIDE SUR LES CHAMPIGNONS ET/OU LES OOMYCETES**
VERWENDUNG EINES ZELLEXTRACTES AUS EINE ODER MEHREREN AMPHIDINIUM MIKROALGEN FÜR DESSEN ANTIFUNGALER WIRKUNG AUF PILZEN UND/ODER OOMYZETEN
USE OF A CELLULAR EXTRACT FROM ONE OR MORE MICROALGAE OF THE GENUS AMPHIDINIUM FOR ITS FUNGICIDAL ACTIVITY ON FUNGI AND/OR OOMYCETES

(30) Priorité: 08.06.2016 FR 1655263
(43) Date de publication de la demande: 17.04.2019
(73) Titulaire: Immunrise Biocontrol France, 33610 Cestas (FR)
(72) Inventeur: THIEBEAULD DE LA CROUEE, Odon, 92340 Bourg La Reine (FR); THOMAS, Yann, 95120 Ermont (FR)
(74) Mandataire: Regimbeau
(86) Numéro de dépôt international: PCT/EP2017/064046
(87) Numéro de publication internationale: WO 2017/211998

(56) Documents cités:
- US-A1- 2010 099 765
- US-B2- 8 815 565
- CAROLINA H POHL ET AL: "Antifungal free fatty acids: A Review", SCIENCE AGAINST MICROBIAL PATHOGENS: COMMUNICATING CURRENT RESEARCH AND TECHNOLOGICAL ADVANCES, 1 January 2011 (2011-01-01), pages 61 - 71, XP055045203, Retrieved from the Internet <URL:http://www.formatex.info/microbiology3/book/61-71.pdf> [retrieved on 20121122]
- WASHIDA K ET AL: "Karatungiols A and B, two novel antimicrobial polyol compounds, from the symbiotic marine dinoflagellate Amphidinium sp", TETRAHEDRON LETTERS, ELSEVIER, AMSTERDAM, NL, vol. 47, no. 15, 10 April 2006 (2006-04-10), pages 2521 - 2525, XP025003878, ISSN: 0040-4039, [retrieved on 20060410], DOI: 10.1016/J.TETLET.2006.02.045
- ECHIGOYA R ET AL: "The structures of five new antifungal and hemolytic amphidinol analogs from Amphidinium carterae collected in New Zealand", HARMFUL ALGAE, ELSEVIER, AMSTERDAM, NL, vol. 4, no. 2, 1 February 2005 (2005-02-01), pages 383 - 389, XP027686712, ISSN: 1568-9883, [retrieved on 20050201]
- GENOVEFFA NUZZO ET AL: "Antifungal Amphidinol 18 and Its 7-Sulfate Derivative from the Marine Dinoflagellate Amphidinium carterae", JOURNAL OF NATURAL PRODUCTS., vol. 77, no. 6, 27 June 2014 (2014-06-27), US, pages 1524 - 1527, XP055400101, ISSN: 0163-3864, DOI: 10.1021/np500275x

## Description

L'invention concerne le domaine des antifongiques et anti-bactéricides des semences.

Après la seconde guerre mondiale et afin de faire face à l'accroissement mondial de la population, l'avènement de la « révolution verte » a été mis en place en faisant appel aux programmes modernes de sélection variétale, à l'irrigation, aux engrais et aux pesticides de synthèse afin de contrôler la fertilité des sols et les organismes pathogènes. Ces différents éléments ont ainsi permis de quasiment tripler la production alimentaire mondiale en l'espace d'une quarantaine d'années. Aujourd'hui, le défi de l'agriculture est de nourrir 9 milliards d'individus à l'horizon 2050 et de continuer à accroître la production par unité de surface tout en tenant compte des ressources de plus en plus limitées et des contraintes croissantes. Par exemple, les pertes potentielles, dues aux stress biotiques et en l'absence de méthodes de protection des cultures, s'élèveraient à plus de la moitié de la production des céréales. Les produits phytosanitaires conventionnels font donc partie intégrante de la protection des cultures dans le monde afin de limiter les pertes. Cependant, ces produits chimiques ont un fort impact négatif sur la santé humaine et l'environnement incitant à utiliser d'autre moyen de lutte contre les maladies infectieuses tels que le bio-contrôle (ensemble des méthodes de protection des végétaux par l'utilisation de mécanisme naturel). L'objet de ce brevet vise à exploiter les algues unicellulaires, issues du phytoplancton, comme source de nouvelles molécules naturelles capables d'agir en tant que « pesticide biologique » en affectant directement la survie des phytopathogènes infectant des cultures d'importances agronomiques majeures, tels que le blé et la vigne.

Le document Washida et al. « Karatungiols A and B, two novel antimicrobial polyol compounds, from the symbiotic marine dinoflagellate Amphidinium sp », TETRAHEDRON LETTERS, vol. 47, n°15, 10 avril 2006 décrit un procédé de purification des composés Karatungiols A et B.

Le document Echigoya et al., HARMFUL ALGAE, vol.4, n°3, 1 février 2005 décrit un procédé de purification des amphidinols 2, 4, 9, 10, 11, 12 et 13.

US 8 815 565B2 divulgue des extraits *d'Amphidinum carterae* contenant les amphidinols 1 à 15.

Le document Genoveffa et al., JOURNAL OF NATURAL PRODUCTS, vol. 77, n°6, 27 juin 2014, décrit un procédé de purification de l'Amphidinol 18.

### Les fusarioses

En Europe, plusieurs maladies sur blé *(Triticum aestivum)* sont responsables de pertes de rendement ou encore d'une dégradation de la qualité sanitaire des grains. Une des plus importantes est la septoriose (*Septoria* spp.). La fusariose est associée à un complexe d'espèces regroupant deux genres de champignons phytopathogènes, Fusarium et Microdochium (1). Ces deux genres englobent environ 19 espèces capables d'induire la fusariose de l'épi de blé. Les espèces les plus fréquentes en Europe sont *F. graminearum, F. culmorum, F. avenaceum, F. poae, M. nivale* et M. *majus.* Le genre *Fusarium* appartient à la division des Ascomycètes et à la famille des Nectriacées. Le genre *Microdochium* appartient à la famille des Tuberculariacées et regroupe deux espèces, *M. nivale* et M. *majus,* provoquant les mêmes symptômes sur épi et sur feuilles que les Fusaria. Plusieurs espèces de fusarium, dont *Fusarium graminearum* est le plus représenté, peuvent se retrouver ensemble à l'échelle de la région, de la parcelle ou sur un même épi formant ainsi le complexe fusarien. La sévérité, l'incidence et la prévalence de chaque espèce varient selon la localisation géographique, les variations climatiques ainsi que les pratiques culturales. La présence sur un même épi de plusieurs de ces espèces est susceptible de modifier leur équilibre et leur dynamique de production de toxines.

La fusariose du blé peut dévaster une culture quelques semaines avant la récolte. Elle peut être associée à la fois à de fortes pertes de rendement (avortement et faible poids des grains), une réduction de leur qualité germinative ou encore une diminution de leur qualité par la présence de toxines dans les grains. En effet, les champignons du genre *Fusarium,* mais pas du genre *Michrodochium,* sont capables de produire des métabolites secondaires toxiques, les mycotoxines, dont la présence augmente l'incidence de la maladie sur les productions agricoles et constitue un problème majeur économique et de santé publique. Les principaux moyens de lutte contre la fusariose regroupent les pratiques culturales, la résistance variétale et la lutte chimique. A l'heure actuelle, peu de variétés de blé sont résistantes à la fusariose. Cependant, il existe des variétés tolérantes possédant des niveaux de résistance partiels limitant les pertes de rendement et l'accumulation des toxines dans les récoltes. Une fois la culture installée, le recours à la lutte chimique est possible mais d'une efficacité limitée. La diversité des agents pathogènes ainsi que leur sensibilité différente aux matières actives complexifient cette lutte. Par exemple, les champignons du genre *Fusarium* sont sensibles aux triazoles alors que les champignons du genre *Microdochium* sont sensibles aux strobilurines.

### Les septorioses

La septoriose est une maladie du blé responsable d'importantes pertes de rendement et qui provoque le plus de pertes économiques dans le monde, notamment dans les régions tempérées humides. Deux principales formes de septorioses peuvent être distinguées : La septoriose des épis (*Phaeosphaeria nodorum*) et la septoriose foliaire (*Mycosphaerella graminicola*). En France, la septoriose des épis est surtout présente dans les zones continentales alors que celle des feuilles l'est principalement dans le nord-ouest et sur les bordures maritimes où le champignon trouve des conditions climatiques favorables à son développement. Les symptômes provoqués par M. *graminicola* apparaissent successivement sous forme de chloroses, taches de couleur vert clair, avant d'évoluer en taches brunâtres appelées nécroses. Ces nécroses finissent par se fondre les unes dans les autres (coalescence). Ensuite, apparaissent sur ces nécroses les pycnides, fructifications noires à peine visibles à l'œil nu. La nuisibilité de la septoriose en termes de pertes de photosynthèse, croissance ou rendement a été étudiée par plusieurs équipes de recherche. Une nuisibilité qualitative exprimant l'impact de la maladie sur la teneur en protéines des grains récoltés peut ainsi être établie.

*M. graminicola* est un champignon hémibiotrophe établissant une première phase biotrophe où l'infection se déroule sur des tissus vivants puis survient la phase nécrotrophe pendant laquelle le champignon exprime des toxines produisant la mort des tissus colonisés. En fonction des conditions environnementales, la reproduction de *M. graminicola* est de nature sexuée (production d'ascospores) ou asexuée (production de pycnidiospores). Les ascospores, disséminées par le vent sur de longues distances participent notamment à la survie du champignon en l'absence de plante hôte et est considéré comme la source principale d'inoculum primaire pour initier la maladie. Les pycnidiospores, quant à eux, sont en majorité produites durant la phase épidémique de la maladie au cours de plusieurs cycles infectieux successifs. Ces spores sont dispersées sur de courtes distances par l'action de l'éclaboussement des gouttes de pluie. La baisse des rendements potentiels est d'autant plus importante que les dernières feuilles sous l'épi impliquées dans le remplissage des grains sont sévèrement touchées par la maladie. Les pertes de rendement imputable à la septoriose ont été évaluées à 1-2 t.ha⁻¹ en moyenne, avec des cas allant jusqu'à 3-3,5 t.ha⁻¹, ce qui représente une diminution de 40 % des rendements.

Les méthodes de lutte pour contrôler *M. graminicola* sont basées sur l'utilisation de fongicide et de cultivars résistants. Cependant, ces dernières années ont vu une perte important de l'efficacité des fongicides due à une forte sélection des agents pathogènes avec, par exemple, une résistance à la famille des strobilurines ainsi qu'une perte récente de l'efficacité des triazoles en champ.

### Les maladies de la vigne

De nos jours , la vigne est cultivée dans le monde entier jouant un rôle central dans l'économie de nombreux pays. Elle est consommée en raisin de table et en jus, mais son exploitation principale repose sur l'industrie du vin. L'Union Européenne est le plus grand producteur de vin au monde et le plus grand exportateur mondial de produits viti - vinicoles. Le secteur rapporte ainsi à l'économie de l'Union Européenne environ 15 milliards d'euros par an (www. ceev.be). En 2010, le vignoble français couvrait près de 865 000 ha, soit près de 3% des terres arables et permet à la France d'être le premier producteur mondial de vin avec 51,1 millions d'hectolitres. La vigne doit faire face à de nombreuses attaques d'agents pathogènes dont les maladies cryptogamiques. Celles-ci sont appelées « maladie du bois » lorsqu'elles touchent les parties lignifiées de la plante, c'est le cas notamment de l'esca, du black dead arm ou de l'eutypiose. Les champignons qui infectent les baies et les parties herbacées de la vigne (feuilles, tiges...) induisent les maladies «cryptogamiques du feuillage» dont font partie la pourriture grise, le black rot, le mildiou et l'oïdium.

### L'esca

Si le mildiou, l'oïdium et la pourriture grise représentent les trois principales maladies cryptogamiques affectant les vignobles à travers le monde, les maladies du bois causées par des agents fongiques deviennent des facteurs limitant de la production de raisins. Les viticulteurs sont actuellement confrontés à deux problèmes majeurs concernant ces maladies du bois: l'absence de méthodes de lutte et une méconnaissance profonde des différents facteurs biotiques et abiotiques.

Les espèces de champignon les plus répandues à travers le monde pour la maladie de l'esca sont les ascomycètes *Diplodia seriata, Diplodia mutila, Neofusicoccum parvum* et *Neofusicoccum luteum.* En France, les espèces les plus isolées sont *Diplodia seriata* et *Botryosphaeria dothidea.* De nombreux autres champignons dont certains pathogènes sont fréquemment isolés des nécroses du bois de plantes atteintes d'esca. C'est le cas d'*Eutypa lata,* agent responsable de l'Eutypiose. Cette maladie se présente sous deux formes : la forme lente et la forme apoplectique. Les symptômes foliaires sont caractéristiques de la forme lente même s'ils peuvent être présents chez la forme apoplectique. La forme lente se caractérise par des colorations foliaires spécifiques : tâches internervaires jaunâtres sur cépages blancs et bordées de rouges sur cépages noirs, les nervures restant vertes. Ces tâches évoluent progressivement vers un brunissement et un dessèchement. Les symptômes foliaires de la forme lente peuvent être visibles une année sur un cep et disparaître l'année suivante. La forme apoplectique est caractérisée par un dessèchement rapide des organes aériens, rameaux, feuilles et grappes d'une partie ou de la totalité du cep de vigne. Ce symptôme se manifeste généralement lorsque les étés sont chauds, entrainant la mort des ceps en quelques jours seulement sans symptômes annonciateurs. La variété des sources d'inoculum et le développement très lent et non visible des champignons dans le bois de vigne rendent très compliquée la mise en oeuvre des méthodes de lutte. De plus, l'évolution de la réglementation des produits phytosanitaires à l'échelle européenne a conduit à l'interdiction de produits chimiques à base d'arsénite de sodium à cause des effets cancérogènes sur l'homme et de la forte toxicité de ces produits sur l'environnement. De nombreuses recherches sont menées à travers le monde pour tester de nouvelles molécules utilisables en pépinière ou au vignoble.

### La pourriture grise

La pourriture grise est une maladie cryptogamique causée par un champignon ascomycète appelé *Botrytis cinerea.* Il appartient à la classe des *Leotiomycetes, à* l'ordre des *Helotiales* et la famille des *Sclerotiniaceae. B. cinerea* est un champignon nécrotrophe capable de coloniser les tissus végétaux sains, déjà infectés, ainsi que les tissus morts (saprophytisme). Sur feuille, les symptômes apparaissent sous forme de tâches brunes avec un feutrage grisâtre sur la face inférieure (fructifications du champignon) qui ont tendance à s'accroître et à envahir tout le limbe. Les grappes peuvent être touchées avant la floraison et se dessécher. Elles sont surtout sensibles au stade de la véraison où il y a développement d'une coloration brune des baies des cépages blancs et l'apparition d'un épais feutrage gris. Les conidies sont disséminées par le vent et pénètrent dans les organes herbacés de façon directe ou par le biais de blessures. C'est pourquoi l'éclatement des baies dû au mildiou favorise les infections par *B. cinerea.* Cette maladie entraîne non seulement des pertes de rendement pouvant aller jusqu'à 40% (Viniflhor, données 2006) mais elle altère également les qualités organoleptiques des vins. Néanmoins, *Botrytis cinerea* est également responsable de la « pourriture noble » nécessaire à l'obtention de certains vins liquoreux.

### Le mildiou

Les deux maladies qui touchent le plus sévèrement les vignobles à l'heure actuelle sont le mildiou et l'oïdium. L'agent responsable du mildiou, l'oomycète *Plasmospora viticola* appartenant à l'ordre péronosporale, est un parasite obligatoire ; pour le maintenir en vie et le multiplier, il est obligatoire de le propager sur des feuilles de vigne en survie. *P. viticola* s'attaque à tous les tissus herbacés de la vigne ainsi qu'aux grappes. Il provoque des défoliations, le brunissement et l'assèchement des baies et des tiges. En l'absence de traitement et dans des conditions climatiques favorables, le mildiou de la vigne peut dévaster jusqu'à 75% de la récolte de la saison.

Le cycle de vie de *P. viticola* comprend une phase sexuée et une phase asexuée. La phase asexuée conduit à la production de spores nécessaires aux infections secondaires et à la dispersion de l'agent pathogène sur une courte distance, tandis que la phase sexuée produit des oospores quiescentes et résistantes au froid permettant le passage de l'hiver et les infections primaires. La première preuve macroscopique de la présence de mildiou dans un vignoble est l'apparition de tâches jaune pâle et irrégulières (tâches d'huile) grossissant sur la face supérieure ou adaxiale, des feuilles. A mesure que la colonisation interne du mycélium avance, le développement de coussins blancs cotonneux sur la face inférieure en correspondance avec les tâches d'huile devient plus important. Dans les infections avancées ces symptômes sont accompagnés de tissus morts bruns. La lutte contre le mildiou s'organise principalement par des mesures préventives par des pulvérisations de fongicides. S'il est possible de stopper une attaque, les dégâts, une fois occasionnés sur les inflorescences et les grappes, sont irrémédiables.

### L'oïdium

L'oïdium de la vigne (*Erysiphe necator*) est un ascomycète biotrophe obligatoire appartenant à l'ordre des Erysiphales. Le champignon colonise la surface de tous les organes verts de la vigne, notamment la face supérieure des feuilles, et se propage sur les baies. Une phase sexuée qui est caractérisée par la production de cléistothèces contenant des ascospores peut alterner avec une phase asexuée conduisant à la formation de conidiophores portant des conidies. Durant la phase hivernale de repos de la vigne, le champignon survit sous forme d'hyphes dans les bourgeons dormants ou de cléistothèces à la surface de la plante. Les spores contenues dans les cléistothèces seront libérées au printemps pour germer à la surface des bourgeons et des jeunes feuilles. Un hyphe primaire se développe ensuite sur la surface de la feuille, puis un réseau mycélien de plus en plus complexe et ramifié tapisse la surface foliaire. Par la suite, des conidiophores se différencient à partir du mycélium constituant le début de l'étape de sporulation et coloniseront d'autres tissus verts de la plante donnant lieu aux infections secondaires.

La présence du mycélium et des conidiophores portant les conidies à la surface des tissus infectés de l'hôte donne une apparence poudreuse de couleur blanche grisâtre. Le feutrage blanc se développe sur les boutons floraux qui se dessèchent. Seules les jeunes baies ayant un taux de sucre <8% sont sensibles à l'oïdium. Toutes les surfaces foliaires peuvent être sensibles à l'infection et ce, quelque soit leur âge. Les jeunes feuilles infectées prennent d'abord une coloration vert foncé puis les feuilles se déforment et deviennent rabougries. La surface supérieure des feuilles peut présenter des tâches de teinte plus claire et chlorotique ressemblant aux tâches d'huile du mildiou. A l'heure actuelle, le principal moyen de lutte contre les maladies qui touchent le plus sévèrement les vignobles est l'utilisation de pesticides et fongicides en grande quantité. La pression sanitaire est donc particulièrement forte en viticulture.

Les traitements fongicides destinés à lutter principalement contre le mildiou et l'oïdium sont appliqués selon un calendrier précis pour prévenir les dommages dus à l'apparition d'une maladie. L'Union Européenne (UE) emploie environ 68 000 tonnes de fongicides par an pour contrôler les maladies de la vigne, ce qui représente 65% des fongicides utilisés dans l'agriculture alors que seulement 3,3 % de la surface agricole utile de l'UE est occupée par la vigne (Eurostat, 2007). Afin de limiter la forte pression des produits chimiques sur l'environnement et la santé, il est nécessaire d'isoler des molécules d'origine naturelle qui joueront un rôle de protection des cultures contre les maladies infectieuses afin de remplacer à terme les produits phytosanitaires chimiques utilisés jusqu'à présent.

### La tavelure du pommier

La tavelure est avec la moniliose et l'oïdium une des principales affections fongiques du pommier (genre Malus). Elle est causée par un champignon ascomycète nommé *Venturia inaequelis,* dont il existe plusieurs milliers de souches, causant des lésions noires ou brunes à la surface des feuilles, des bourgeons ou des fruits et parfois même sur le bois. Les fruits et la partie inférieure des feuilles y sont spécialement sensibles.

Le champignon hiverne sur les feuilles qui tombent des arbres infectés, sous la forme de périthèces. Au printemps, au moment de l'éclosion des bourgeons, les périthèces se remplissent d'ascospores. Les ascospores sont éjectées dans l'air du verger lors des journées humides et atteignent les arbres grâce aux déplacements d'air. Cette décharge d'ascospores commence au débourrement et se poursuit pendant 6 à 10 semaines, le plus souvent jusqu'à la fin juin. Lorsque les ascospores atteignent le feuillage et que les feuilles sont mouillées pendant un certain temps, ils germent et pénètrent les feuilles: il y a alors infection primaire. Selon les conditions d'humidité et de température, l'infection fongique devient visible en une à trois semaines sur les différentes parties de l'arbre. Des taches olive foncé ou brunes d'environ 5 mm apparaissent sur les feuilles et peuvent éventuellement couvrir toute la feuille. Les fleurs infectées peuvent tomber. L'infection des fruits se reconnaît d'abord par des taches grises au niveau de la tige.

Suite à l'infection primaire et pour le reste de l'été, le champignon se développe et engendre des conidies qui sont une autre forme de structure reproductrice. Lorsque les conidies s'échappent, il y a infection secondaire. Les conidies peuvent infecter n'importe quelle partie de l'arbre et celles produites en fin d'été peuvent même se développer sur les fruits entreposés. La pluie forte se charge de disperser les conidies.

La maladie tue rarement son hôte mais peut réduire significativement (jusqu'à 100 %) la qualité et la production des fruits en l'absence de traitement par fongicide. Après les moyens préventifs qui consistent à ramasser les feuilles tombées lors de l'automne, la stratégie de lutte impose d'agir efficacement au printemps afin d'éviter que les spores relâchées n'infectent ou ne puissent se développer sur les arbres. La méthode traditionnelle de protection consistait à commencer l'application de fongicide dès le débourrement et de répéter les traitements tous les sept jours environ jusqu'à la fin juin afin de protéger les nouvelles pousses. Les vergers de pommier sont les plus traités en fongicides et en insecticides avec une moyenne de 28,8 traitements fongicides par an, dont 19 sont dédiés à la tavelure (données INRA).

### Les micro-algues

Les molécules d'origine naturelle ayant un nouveau mécanisme d'action et capable de contourner les résistances développées par les agents pathogènes ont un devenir majeur pour l'élaboration de nouveaux produits phytosanitaires respectueux de l'environnement. Les océans représentent une variété considérable d'organismes (bactéries, microalgues, algues, animaux vertébrés et invertébrés) qui sont une source de nouvelles molécules bioactives et qui sont encore peu exploités (2). Par exemple, les micro-organismes marins accumulent des métabolites secondaires bioactifs dont leur structure unique n'est pas retrouvée chez les organismes terrestres. Ces métabolites représentent donc potentiellement de nouvelles molécules d'intérêt. Certaines substances issues d'organismes marins ont été décrits comme possédant une activité antifongique ou une activité de substance naturelle de défense, mais la recherche de ces molécules est encore très peu développée (3).

Les micro-algues sont des organismes unicellulaires jouant un rôle clé dans les écosystèmes aquatiques. Produisant du matériel organique, ils jouent un rôle écologique important car ils représentent la base de la chaine alimentaire du milieu marin. Cependant, leur incroyable capacité à coloniser l'ensemble des océans du globe suggère qu'elles ont probablement développé des stratégies de lutte efficaces contre les agents pathogènes via notamment la production de pesticides naturels. Par exemple, l'abondante prolifération dans les régions côtières des micro-algues produisant des biotoxines est responsable de la formation des bloom toxiques d'algue (HABs: Harmful algal blooms) ayant une conséquence importante sur la cascade trophique.

Parmi les micro-algues, les dinoflagellés, appartenant à l'ordre des Gymnodiniales et à la famille des Gymnodiniacées sont présents dans les eaux marines tempérées et tropicales vivant sous forme libres ou en symbiose avec les invertébrés (par exemple, les coraux). Les dinoflagellés synthétisent un nombre important de métabolites secondaires de type polycétides (composés ayant une activité biologique ou pharmacologique pouvant être toxique afin de conférer un avantage pour la survie) dont plusieurs ont été caractérisés dont ceux responsables des HAB (4). Par exemple, l'espèce modèle des dinoflagellés, *Amphidinium carterae,* produit une profusion de différents composés bioactifs dont plusieurs ont le devenir d'être développés en tant qu'agents thérapeutiques (5). Les polycétides produits par les espèces d'Amphidinium sont extrêmement diverses en structure et forment trois catégories : les macrolides, les polycétides lineaires et les polycétides à longue chaine. Par exemple, les amphidinols sont des polyhydroxy-polyenes (ploycétides à longue chaine) qui présentent une forte activité anti-fongique et hémolytique. Ils augmentent ainsi la perméabilité membranaire en s'associant avec les lipides membranaires (6). Parmi les différentes souches d'Amphidinium, des composées similaires aux amphidinols ayant une longue chaîne polyhydroxy ont été isolés tels que les lingshuiols, karatungiols, carteraol E, luteophanols, colopsinols, et amphezonol A (5).

Afin de limiter la forte pression des produits chimiques sur l'environnement et la santé, il est nécessaire d'isoler des molécules d'origine naturelle qui joueront un rôle de protection des cultures contre les maladies infectieuses afin de remplacer à terme les produits phytosanitaires chimiques utilisés jusqu'à présent. Ces « pesticides biologiques » pourraient ainsi affecter directement la survie des phytopathogènes des cultures d'importances agronomiques majeures, tels que le blé et la vigne.

De façon surprenante, les inventeurs ont observé un effet fongicide d'un extrait cellulaire *d'Amphidinium carterae,* sur de nombreux champignons pathogènes des plantes.

### RESUME DE L'INVENTION

L'objet de l'invention concerne un procédé de lutte contre les champignons et/ou les oomycètes des plantes et semence de culture caractérisé par les étapes suivantes :
- Récolte de cellules fraiches d'une ou plusieurs micro-algue(s) du genre *Amphidinium ;*
- Congélation et/ou lyophilisation desdites cellules ;
- Suspension dudit lyophilisat ou desdites cellules congelées dans de l'eau, dans un rapport en poids de 1:200 à 1:2 à une température supérieure à 60°C ;
- Application sur des plantes de culture et/ou enrobage de semences de l'extrait ainsi obtenu.

### LEGENDE DES FIGURES

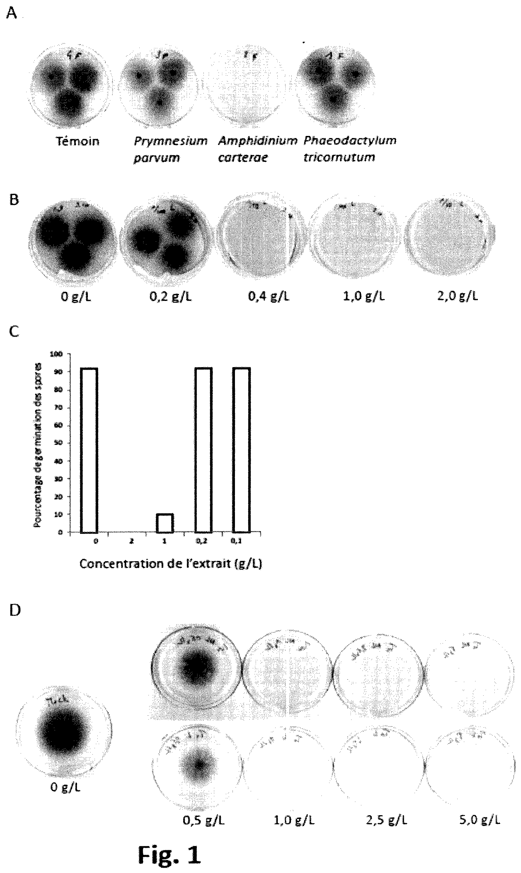

**Figure 1****. L'extrait** ***d'Amphidinium** carterae* **possède une activité anti-fongique sur *Fusarium graminearum.***
   A. Des spores de *Fusarium graminearum* ont été incubées en présence d'extraits provenant de différentes cultures de micro-algues puis mises sur milieu de culture *in vitro.* La photo a été prise 72H après.
   B. Des spores de *Fusarium graminearum* ont été incubés en présence d'une gamme de concentration d'extraits (de 0 g/L à 2,0 g/L) provenant d'une culture *d'Amphidinium carterae* puis mises sur milieu de culture *in vitro.* La photo a été prise 72H après.
   C. Similaire à B mais la quantité de spores germées est dénombrée 6H après l'incubation.
   D. Des spores de *Fusarium graminearum* ont été incubées en présence d'extraits d'A. *carterae* qui ont été préalablement congelés (Figure 1 D en haut) ou lyophilisés (Figure 1 D en bas).

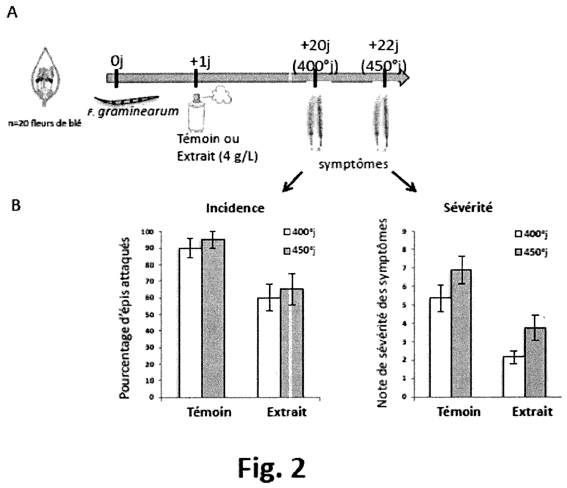

**Figure 2****. L'extrait *d'Amphidinium carterae* inhibe *in vitro* et *in planta* la croissance de champignons pathogènes du blé.**
   A. Mode opératoire d'inoculation des fleurs de blé avec des spores de Fusarium graminearum puis traités 24h après avec une solution en présence ou non (mock) de l'extrait *d'Amphidinium carterae.*
   **B.** L'apparition et le développement des symptômes sont suivis et les niveaux d'incidence et de sévérité (note de 0 à 9) sont notés à 400°J et à 450°J.

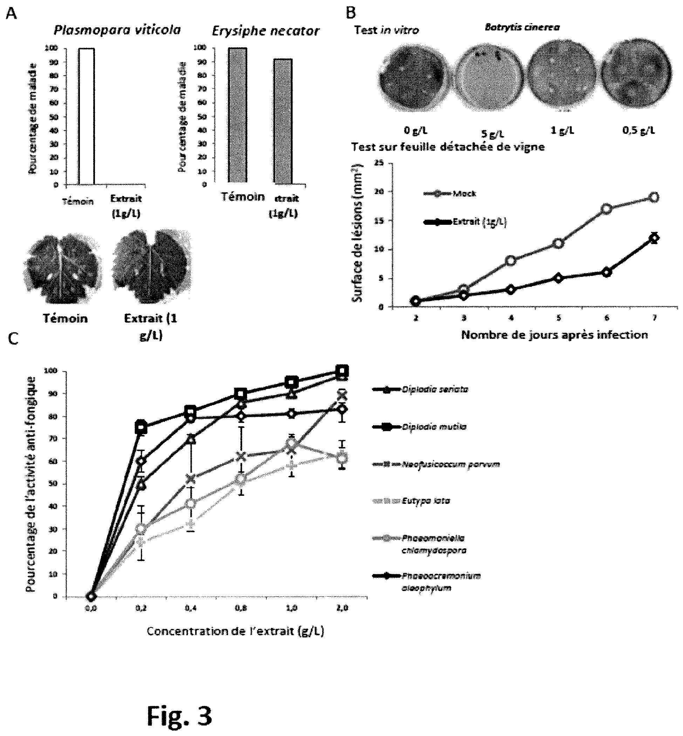

**Figure 3****. L'extrait *d'Amphidinium carterae* inhibe *in vitro* et *in planta* la croissance de champignons pathogènes de la vigne**
   A. L'extrait *d'Amphidinium carterae* (1 g/L) ou de l'eau stérile (mock) ont été pulvérisés sur des feuilles détachées de vigne maintenues en condition *in vitro* puis des sporanges de *Plasmopara viticola* ou des conidies *d'Erysiphe necator* ont été déposées sur ces feuilles. La lecture des symptômes est réalisée à 7 jours et à 12 jours respectivement.
   **B.** Similaire à A mais des implants mycéliens de *Botrytis cinerea* ont été déposés sur les feuilles traitées. La lecture des symptômes s'est réalisée à 7 jours par une mesure de la taille des nécroses.
   **C.** Des implants mycéliens de différents champignons impliqués dans la maladie de l'esca de la vigne ont été déposés sur un milieu de culture puis traités 24H après avec de l'eau (mock) ou de l'extrait d'A. *carterae* à différentes concentrations. La lecture des symptômes se fait par la mesure de la surface du mycélium à 4 jours.

### DESCRIPTION DETAILLEE DE L'INVENTION

L'objet de l'invention concerne un procédé de lutte contre les champignons et/ou les oomycètes des plantes et semence de culture caractérisé par les étapes suivantes :
- Récolte de cellules fraiches d'une ou plusieurs micro-algue(s) du genre *Amphidinium ;*
- Congélation et/ou lyophilisation desdites cellules ;
- Suspension dudit lyophilisat ou desdites cellules congelées dans de l'eau, dans un rapport en poids de 1:200 à 1:2 à une température supérieure à 60°C ;
- Application sur des plantes de culture et/ou enrobage de semences de l'extrait ainsi obtenu.

### Les Amphidinium

Des *Amphidinium* appropriés sont choisis dans le groupe constitué de *Amphidinium achromaticum, Amphidinium aculeatum, Amphidinium acutissimum, Amphidinium acutum, Amphidinium alinii, Amphidinium aloxalocium, Amphidinium amphidinioides, Amphidinium asymmetricum, Amphidinium aureum, Amphidinium belauense, Amphidinium bidentatum, Amphidinium bipes, Amphidinium boekhoutensis, Amphidinium boggayum, Amphidinium caerulescens, Amphidinium carbunculus, Amphidinium carterae, Amphidinium celestinum, Amphidinium chattonii, Amphidinium coeruleum, Amphidinium conradii, Amphidinium conus, Amphidinium coprosum, Amphidinium corallinum, Amphidinium corpulentum, Amphidinium crassum, Amphidinium cristatum, Amphidinium cucurbita, Amphidinium cucurbitella, Amphidinium cupulatisquama, Amphidinium curvatum, Amphidinium cyaneoturbo, Amphidinium dentatum, Amphidinium discoidale, Amphidinium dubium, Amphidinium eilatiensis, Amphidinium emarginatum, Amphidinium fastigium, Amphidinium filum Böhm, Amphidinium flagellans, Amphidinium flexum, Amphidinium galbanum, Amphidinium gibbosum, Amphidinium glaucovirescens, Amphidinium glaucum, Amphidinium globosum, Amphidinium hadai, Amphidinium herdmanii, Amphidinium incoloratum, Amphidinium inflatum, Amphidinium kesselitzii, Amphidinium kesslitzii, Amphidinium klebsii, Amphidinium lacunarum, Amphidinium lanceolatum, Amphidinium lefevrei, Amphidinium lilloense, Amphidinium lissae, Amphidinium longum, Amphidinium luteum, Amphidinium machapungarum, Amphidinium macrocephalum, Amphidinium mammillatum, Amphidinium manannini, Amphidinium mananninii, Amphidinium massartii, Amphidinium mootonorum, Amphidinium mucicola, Amphidinium nasutum, Amphidinium obliquum, Amphidinium obrae, Amphidinium oceanicum, Amphidinium oculatum, Amphidinium operculatum, Amphidinium operculatum var. steinii, Amphidinium ornithocephalum, Amphidinium ovoideum, Amphidinium ovum, Amphidinium pacificum, Amphidinium pelagicum, Amphidinium phthartum, Amphidinium psammophila, Amphidinium psittacus, Amphidinium purpureum, Amphidinium pusillum, Amphidinium rhynchocephalum, Amphidinium roseolum, Amphidinium ruttneri, Amphidinium salinum, Amphidinium schilleri, Amphidinium schroederi, Amphidinium scissum, Amphidinium sphagnicola, Amphidinium sphenoides, Amphidinium steinii, Amphidinium stellatum, Amphidinium stigmatum, Amphidinium sulcatum, Amphidinium tortum, Amphidinium trochodinioides, Amphidinium trochodinoides, Amphidinium trulla, Amphidinium truncatum, Amphidinium turbo, Amphidinium vernal, Amphidinium vigrense, Amphidinium vitreum, Amphidinium vittatum, Amphidinium wigrense, Amphidinium yoorugurrum, Amphidinium yuroogurrum.*

De préférence, la ou l'une des micro-algues du genre *Amphidinium* utilisée(s) selon l'invention est *Amphidinium carterae.* Il existe plusieurs souches *d'Amphidinium carterae* en collection comme les souches CCMP 124, 1314, 3177 (CCMP = Culture Collection of Marine Phytoplankton), AC 208, 792 (AC =Algobank Cean ), BEA 01198 (BEA= Banco Español de Algas).

Avantageusement, la souche *d'Amphidinium carterae* utilisée selon l'invention est CCMP 1314, AC208 ou AC792.

De préférence, ledit extrait comprend l'amphidinol 18 ou l'amphidinol 19, de manière particulièrement préférée l'amphidinol 18, avantageusement en une quantité supérieure à 1% p/p par rapport au poids total de l'extrait, de préférence comprise entre 2 et 10% p/p par rapport au poids total de l'extrait, de manière particulièrement préférée comprise entre 3 et 5% p/p par rapport au poids total de l'extrait.

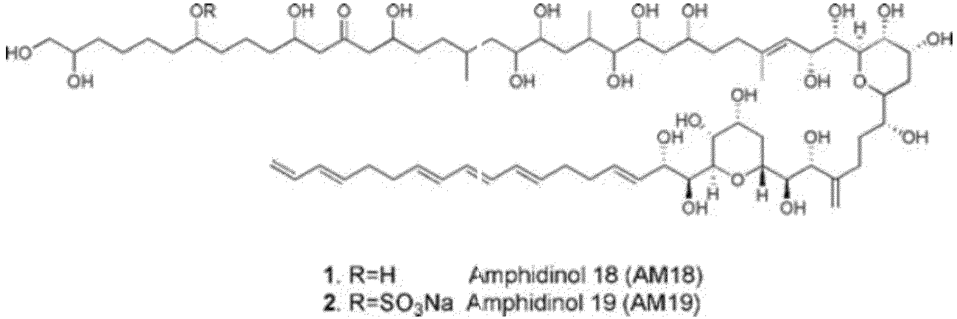

### Mode d'action

Cette activité fongicide sur les champignons et/ou les oomycètes pathogènes des plantes et semences de culture peut en particulier s'exercer par inhibition de la germination des spores ou par inhibition de la croissance du champignon et/ou des oomycètes.

L'activité s'exerce par une activité lytique des parois et membranes cellulaires qui aboutit à la lyse cellulaire.

### Plantes de culture

Lesdites plantes de culture sont en particulier choisies dans le groupe constitué des céréales comme le blé, maïs, orge, riz, soja, des fruits et légumes comme pomme de terre, carotte, pommiers, pêchers, abricotiers, tomates, radis, haricots, de la vigne et des plantes d'ornement.

Lesdites plantes de culture sont en particulier choisies dans le groupe constitué des genres *Abelmoschus, Acacia, Achras, Agave, Agrostis, Aleurites, Allium, Anacardium, Ananas, Annona, Apium, Arachis, Areca, Armoracia, Arracacia, Artocarpus, Asparagus, Aspidosperma, Avena, Bertholletia, Beta, Boehmeria, Borassus, Brassica, Cajanus, Camellia, Cannabis, Capsicum, Carica, Carthamus, Carum, Carya, Castanea, Ceiba, Ceratonia, Chenopodium, Chrysanthemum, Cicer, Cichorium, Cinchona, Cinnamomum, Citrullus, Citrus, Cocos, Coffea, Cola, Colocasia, Corchorus, Corylus, Crotalaria, Cucumis, Cucurbita, Cydonia, Cymbopogon, Cynara, Dactylis, Daucus, Dioscorea, Diospyros, Echinochloa, Elaeis, Elettaria, Eleusine, Eragrostis, Eriobotrya, Eugenia, Fagopyrum, Ficus, Foeniculum, Fragaria, Furcraea, Glycine, Glycyrrhiza, Gossypium, Guizotia, Helianthus, Hevea, Hibiscus, Hordeum, Humulus, Ilex, Indigofera, Ipomoea, Jasminum, Juglans, Lactuca, Lagenaria, Lavandula, Lawsonia, Lens, Lepidium, Lespedeza, Linum, Litchi, Lolium, Lopmoea, Lotus, Lupinus, Lycopersicon, Lygeum, Macadamia, Malus, Mangifera, Manihot, Maranta, Medicago, Mentha, Mespilus, Metroxylon, Moringa, Musa, Myristica, Nicotiana, Olea, Onobrychis, Oryza, Panicum, Papaver, Pastinaca, Pelargonium, Pennisetum, Persea, Phaseolus, Phleum, Phoenix, Phormium, Pimpinella, Piper, Pistacia, Pisum, Prunus, Psidium, Punica, Pyrus, Raphanus Rheum, Ribes, Ricinus, Rose, Rubus, Saccharum, Scorzonera, Secale Sechium, Sesamum, Setaria, Solanum, Sorghum, Spinacia, Theobroma, Tragopogon, Trifolium, Trigonella, Triticum, Urena, Vaccinium, Valerianella, Vanilla, Vicia, Vigna, Vitellaria, Vitis, Xanthosoma, Zea, Zingiber.*

### Pathogènes

Lesdits champignons pathogènes des plantes et semences de culture sont des ascomycètes ou des basidiomycètes, de préférence des ascomycètes.

Lesdits champignons pathogènes des plantes et semences de culture sont des champignons pathogènes des plantes et semences de culture des genres :
Acrocalymma, *Acrocalymma medicaginis,*
Fusarium, *Fusarium affine, Fusarium arthrosporioides, Fusarium crookwellense, Fusarium culmorum, Fusarium graminearum, Fusarium moniliforme, Fusarium incarnatum, Fusarium solani, Fusarium langsethiae, Fusarium mangiferae, Fusarium oxysporum f.sp. albedinis, Fusarium oxysporum f.sp. asparagi, Fusarium oxysporum f.sp. batatas, Fusarium oxysporum f.sp. betae, Fusarium oxysporum f.sp. cannabis, Fusarium oxysporum f.sp. carthami, Fusarium oxysporum f.sp. cattleyae, Fusarium oxysporum f.sp. ciceris, Fusarium oxysporum f.sp. coffea, Fusarium oxysporum f.sp. cubense, Fusarium oxysporum f.sp. cyclaminis, Fusarium oxysporum f.sp. dianthi, Fusarium oxysporum f.sp. lentis, Fusarium oxysporum f.sp. lini, Fusarium oxysporum f.sp. lycopersici, Fusarium oxysporum f.sp. medicaginis, Fusarium oxysporum f.sp. pisi, Fusarium oxysporum f.sp. radicis-lycopersici, Fusarium oxysporum f.sp. spinacia, Fusarium oxysporum, Fusarium pallidoroseum, Fusarium patch, Fusarium proliferatum, Fusarium redolens, Fusarium sacchari, Fusarium solani, Fusarium subglutinans, Fusarium sulphureum, Fusarium tricinctum, Fusarium wilt,*
Botrytis, *Botrytis allii, Botrytis anthophila, Botrytis cinerea, Botrytis fabae, Botrytis narcissicola,*
Alternaria, *Alternaria alternata, Alternaria brassicae, Alternaria brassicicola, Alternaria carthami, Alternaria cinerariae, Alternaria dauci, Alternaria dianthi, Alternaria dianthicola, Alternaria euphorbiicola, Alternaria helianthi, Alternaria helianthicola, Alternaria japonica, Alternaria leucanthemi, Alternaria limicola, Alternaria linicola, Alternaria padwickii, Alternaria panax, Alternaria radicina, Alternaria raphani, Alternaria saponariae, Alternaria senecionis, Alternaria solani, Alternaria tenuissima, Alternaria triticina, Alternaria zinniae,*
Erisyphe, *Erisyphe necator, Erysiphe betae, Erysiphe brunneopunctata, Erysiphe cichoracearum, Erysiphe cruciferarum, Erysiphe graminis f. sp. Avenae, Erysiphe graminis f.sp. tritici, Erysiphe heraclei, Erysiphe pisi,*
Claviceps, *Claviceps fusiformis, Claviceps purpurea, Claviceps sorghi, Claviceps zizaniae,*
Gaeumannomyces, *Gaeumannomyces graminis,*
Leptosphaeria, *Leptosphaeria nodorum, Leptosphaeria acuta, Leptosphaeria cannabina, Leptosphaeria coniothyrium, Leptosphaeria libanotis, Leptosphaeria lindquistii, Leptosphaeria maculons, Leptosphaeria musarum, Leptosphaeria pratensis, Leptosphaeria sacchari, Leptosphaeria woroninii,*
Microdochium, *Microdochium spp. Microdochium bolleyi, Microdochium dimerum, Microdochium panattonianum, Microdochium phragmitis,*
Mycosphaerella, *Mycosphaerella arachidis, Mycosphaerella areola, Mycosphaerella berkeleyi, Mycosphaerella bolleana, Mycosphaerella brassicicola, Mycosphaerella caricae, Mycosphaerella caryigena, Mycosphaerella cerasella, Mycosphaerella coffeicola, Mycosphaerella confusa, Mycosphaerella cruenta, Mycosphaerella dendroides, Mycosphaerella eumusae, Mycosphaerella gossypina, Mycosphaerella graminicola, Mycosphaerella henningsii, Mycosphaerella horii, Mycosphaerella juglandis, Mycosphaerella lageniformis, Mycosphaerella linicola, Mycosphaerella louisianae, Mycosphaerella musae, Mycosphaerella musicola, Mycosphaerella palmicola, Mycosphaerella pinodes, Mycosphaerella pistaciarum, Mycosphaerella pistacina, Mycosphaerella platanifolia, Mycosphaerella polymorpha, Mycosphaerella pomi, Mycosphaerella punctiformis, Mycosphaerella pyri,*
Oculimacula, *Oculimacula acuformis, Oculimacula yallundae,*
Blumeria, *Blumeria graminis,*
Pyrenophora, *Pyrenophora avenae, Pyrenophora chaetomioides, Pyrenophora graminea, Pyrenophora seminiperda, Pyrenophora teres, Pyrenophora teres f. maculata, Pyrenophora teres f. teres, Pyrenophora tritici-repentis,*
Ramularia, *Ramularia collo-cygni, Ramularia beticola, Ramularia coryli, Ramularia cyclaminicola, Ramularia macrospora, Ramularia menthicola, Ramularia necator, Ramularia primulae, Ramularia spinaciae, Ramularia subtilis, Ramularia tenella, Ramularia vallisumbrosae,*
Rhynchosporium, *Rhynchosporium secalis,*
Cochliobolus, *Cochliobolus, Cochliobolus carbonum, Cochliobolus cymbopogonis, Cochliobolus hawaiiensis, Cochliobolus heterostrophus, Cochliobolus lunatus, Cochliobolus miyabeanus, Cochliobolus ravenelii, Cochliobolus sativus, Cochliobolus setariae, Cochliobolus spicifer, Cochliobolus stenospilus, Cochliobolus tuberculatus, Cochliobolus victoriae,*
Microdochium, *Microdochium oryzae,*
Pyricularia, *Pyricularia oryzae,*
Sarocladium, *Sarocladium oryzae,*
Ustilaginoides, *Ustilaginoides virens,*
Cercospora, *Cercospora, Cercospora apii, Cercospora apii f.sp. clerodendri, Cercospora apiicola, Cercospora arachidicola, Cercospora asparagi, Cercospora atrofiliformis, Cercospora beticola, Cercospora brachypus, Cercospora brassicicola, Cercospora brunkii, Cercospora cannabis, Cercospora cantuariensis, Cercospora capsici, Cercospora carotae, Cercospora corylina, Cercospora fuchsiae, Cercospora fusca, Cercospora fusimaculans, Cercospora gerberae, Cercospora halstedii, Cercospora handelii, Cercospora hayi, Cercospora hydrangeae, Cercospora kikuchii, Cercospora lentis, Cercospora liquidambaris, Cercospora longipes, Cercospora longissima, Cercospora mamaonis, Cercospora mangiferae, Cercospora medicaginis, Cercospora melongenae, Cercospora minuta, Cercospora nicotianae, Cercospora odontoglossi, Cercospora papayae, Cercospora penniseti, Cercospora pisa-sativae, Cercospora platanicola, Cercospora puderii, Cercospora pulcherrima, Cercospora rhapidicola, Cercospora rosicola, Cercospora sojina, Cercospora solani, Cercospora solani-tuberosi, Cercospora sorghi, Cercospora theae, Cercospora tuberculans, Cercospora vexans, Cercospora vicosae, Cercospora zeae-maydis, Cercospora zebrina, Cercospora zonata,*
Corynespora, *Corynespora cassiicola,*
Phakospora, *Phakospora pachyrhizi, Phakopsora gossypii, Colletotrichum, Colletotrichum acutatum, Colletotrichum arachidis, Colletotrichum capsici, Colletotrichum cereale, Colletotrichum coffeanum, Colletotrichum crassipes, Colletotrichum dematium, Colletotrichum dematium f. spinaciae, Colletotrichum derridis, Colletotrichum destructivum, Colletotrichum gloeosporioides, Colletotrichum glycines, Colletotrichum gossypii, Colletotrichum graminicola, Colletotrichum higginsianum, Colletotrichum kahawae, Colletotrichum lindemuthianum, Colletotrichum lini, Colletotrichum mangenotii, Colletotrichum musae, Colletotrichum nigrum, Colletotrichum orbiculare, Colletotrichum pisi, Colletotrichum sublineolum, Colletotrichum trichellum, Colletotrichum trifolii, Colletotrichum truncatum,*
*Pythium spp.,*
Diplodia, *Diplodia allocellula, Diplodia laelio-cattleyae, Diplodia manihoti, Diplodia paraphysaria, Diplodia seriata, Diplodia theae-sinensis,*
Monilia, *Monilinia azaleae, Monilinia fructicola, Monilinia fructigena, Monilinia laxa, Monilinia oxycocci,*
Pezzicula, *Pezzicula alba, Pezzicula malicorticis,*
Zymoseptoria, *Zymoseptoria tritici*
Phytophthora, *Phytophthora infestans*
Guignardia, *Guignardia bidwelli, Guignardia camelliae, Guignardia fulvida, Guignardia mangiferae, Guignardia musae, Guignardia philoprina,*
Plasmopara, *Plasmopara viticola,*
Puccinia, *Puccinia angustata, Puccinia arachidis, Puccinia aristidae, Puccinia asparagi, Puccinia cacabata, Puccinia campanulae, Puccinia carthami, Puccinia coronata, Puccinia dioicae, Puccinia erianthi, Puccinia extensicola, Puccinia helianthi, Puccinia hordei, Puccinia jaceae, Puccinia kuehnii, Puccinia malvacearum, Puccinia mariae-wilsoniae, Puccinia melanocephala, Puccinia menthae, Puccinia oxalidis, Puccinia pelargonii-zonalis, Puccinia pittieriana, Puccinia poarum, Puccinia purpurea, Puccinia recondita, Puccinia schedonnardii, Puccinia sessilis, Puccinia striiformis, Puccinia striiformis, Puccinia subnitens, Puccinia substriata, Puccinia verruca, Puccinia xanthii,*
Rhizoctonia, *Rhizoctonia solani, Rhizoctonia oryzae, Rhizoctonia cerealis, Rhizoctonia leguminicola, Rhizoctonia rubi,*
Sclerotinia, *Sclerotinia borealis, Sclerotinia bulborum, Sclerotinia minor, Sclerotinia ricini, Sclerotinia sclerotiorum, Sclerotinia spermophila, Sclerotinia trifoliorum,*
Septoria, *Septoria ampelina, Septoria azaleae, Septoria bataticola, Septoria campanulae, Septoria cannabis, Septoria cucurbitacearum, Septoria darrowii, Septoria dianthi, Septoria eumusae, Septoria glycines, Septoria helianthi, Septoria humuli, Septoria hydrangeae, Septoria lactucae, Septoria lycopersici, Septoria lycopersici, Septoria menthae, Septoria passerinii, Septoria pisi, Septoria rhododendri, Septoria secalis, Septoria selenophomoides,*
Venturia, *Venturia inaequalis. Venturia carpophila,*
Acrodontium, *Acrodontium simplex,*
Acrophialophora, *Acrophialophora fusispora,*
Acrosporium, *Acrosporium tingitaninum,*
Aecidium, *Aecidium aechmantherae, Aecidium amaryllidis, Aecidium breyniae, Aecidium campanulastri, Aecidium cannabis, Aecidium cantensis, Aecidium caspicum, Aecidium foeniculi, Aecidium narcissi,*
Ahmadiago,
Albonectria, *Albonectria rigidiuscula,*
*Allodus, Allodus podophylli,*
*Amphobotrys, Amphobotrys ricini,*
*Anguillosporella, Anguillosporella vermiformis,*
*Anthostomella, Anthostomella pullulans,*
*Antrodia, Antrodia albida, Antrodia serialiformis, Antrodia serialis,*
*Apiospora, Apiospora montagnei,*
*Appendiculella,*
*Armillaria Armillaria heimii, Armillaria sinapina, Armillaria socialis, Armillaria tabescens,*
Arthrocladiella,
Arthuriomyces, *Arthuriomyces peckianus,*
Ascochyta, *Ascochyta asparagina, Ascochyta bohemica, Ascochyta caricae, Ascochyta doronici, Ascochyta fabae f.sp. lentis, Ascochyta graminea, Ascochyta hordei, Ascochyta humuli, Ascochyta pisi, Ascochyta prasadii, Ascochyta sorghi, Ascochyta spinaciae, Ascochyta tarda, Ascochyta tritici,*
Ascospora, *Ascospora ruborum,*
Aspergillus, *Aspergillus aculeatus, Aspergillus fischerianus, Aspergillus niger,*
Asperisporium, *Asperisporium caricae,*
*Asteridiella,*
Asteroma, *Asteroma caryae,*
Athelia, *Athelia arachnoidea, Athelia rolfsii,*
Aurantiporus, *Aurantiporus fissilis,*
Aureobasidium, *Aureobasidium pullulans,*
*Bambusiomyces,*
*Banana freckle,*
*Bayoud disease,*
Beniowskia, *Beniowskia sphaeroidea,*
Bionectria, *Bionectria ochroleuca,*
Bipolaris, *Bipolaris cactivora, Bipolaris cookei, Bipolaris incurvata, Bipolaris sacchari,*
Biscogniauxia, *Biscogniauxia capnodes, Biscogniauxia marginata,*
Bjerkandera, *Bjerkandera adusta,*
*Block sigatoka,*
Blakeslea, *Blakeslea trispora,*
Botryodiplodia, *Botryodiplodia oncidii, Botryodiplodia ulmicola,*
Botryosphaeria, *Botryosphaeria cocogena, Botryosphaeria dothidea, Botryosphaeria marconii, Botryosphaeria obtusa, Botryosphaeria rhodina, Botryosphaeria ribis, Botryosphaeria stevensii,*
Botryosporium, *Botryosporium pulchrum,*
Botryotinia, *Botryotinia fuckeliana, Botryotinia polyblastis,*
*Boxwood blight,*
*Brachybasidiaceae,*
Brasiliomyces, *Brasiliomyces malachrae,*
Briosia, *Briosia ampelophaga,*
*Brown ring patch,*
*Buckeye rot of tomato,*
*Bulbomicrosphaera,*
Cadophora, *Cadophora malorum,*
*Caespitotheca,*
Calonectria, *Calonectria ilicicola, Calonectria indusiata, Calonectria kyotensis, Calonectria pyrochroa, Calonectria quinqueseptata,*
Camarotella, *Camarotella acrocomiae, Camarotella costaricensis,*
*Canna rust,*
Capitorostrum, *Capitorostrum cocoes,*
Capnodium, *Capnodium footii, Capnodium mangiferum, Capnodium ramosum, Capnodium theae,*
Cephalosporium, *Cephalosporium gramineum,*
Ceratobasidium, *Ceratobasidium cereale, Ceratobasidium cornigerum, Ceratobasidium noxium, Ceratobasidium ramicola, Ceratobasidium setariae, Ceratobasidium stevensii,*
Ceratocystis, *Ceratocystis adiposa, Ceratocystis coerulescens, Ceratocystis fimbriata, Ceratocystis moniliformis, Ceratocystis oblonga, Ceratocystis obpyriformis, Ceratocystis paradoxa, Ceratocystis pilifera, Ceratocystis pluriannulata, Ceratocystis polyconidia, Ceratocystis tanganyicensis, Ceratocystis zombamontana,*
Ceratorhiza, *Ceratorhiza hydrophila,*
*Ceratospermopsis,*
Cercoseptoria, *Cercoseptoria ocellata,*
Cercosporella, *Cercosporella rubi,*
Ceriporia, *Ceriporia spissa, Ceriporia xylostromatoides,*
Cerrena, *Cerrena unicolor,*
Ceuthospora, *Ceuthospora lauri,*
Choanephora, *Choanephora cucurbitarum, Choanephora infundibulifera,*
Chrysanthemum, *Chrysanthemum white rust,*
Chrysomyxa, *Chrysomyxa cassandrae,*
Chrysomyxa, *Chrysomyxa himalensis, Chrysomyxa ledi, Chrysomyxa ledi var. rhododendri, Chrysomyxa ledicola, Chrysomyxa nagodhii, Chrysomyxa neoglandulosi, Chrysomyxa piperiana, Chrysomyxa pirolata, Chrysomyxa pyrolae, Chrysomyxa reticulata, Chrysomyxa roanensis, Chrysomyxa succinea,*
Cladosporium, *Cladosporium arthropodii, Cladosporium cladosporioides, Cladosporium cladosporioides f.sp. pisicola, Cladosporium cucumerinum, Cladosporium herbarum, Cladosporium musae, Cladosporium oncobae,*
Climacodon, *Climacodon pulcherrimus, Climacodon septentrionalis,*
Clitocybe, *Clitocybe parasitica,*
*Clonostachys rosea f. rosea,*
Clypeoporthe, *Clypeoporthe iliau,*
Coleosporium, *Coleosporium helianthi, Coleosporium ipomoeae, Coleosporium madiae, Coleosporium pacificum, Coleosporium tussilaginis,*
*Conidiosporomyces,*
Coniella, *Coniella castaneicola, Coniella diplodiella, Coniella fragariae,*
Coniothecium, *Coniothecium chomatosporum,*
Coniothyrium, *Coniothyrium celtidis-australis, Coniothyrium henriquesii, Coniothyrium rosarum, Coniothyrium wernsdorffiae,*
Coprinopsis, *Coprinopsis psychromorbida,*
Cordana, *Cordana johnstonii, Cordana musae, Coriolopsis floccosa,*
*Corn grey leaf spot,*
Corticium, *Corticium invisum, Corticium penicillatum, Corticium theae,*
Coryneopsis, *Coryneopsis rubi,*
Coryneum, *Coryneum rhododendri,*
*Covered smut,*
Crinipellis, *Crinipellis sarmentosa,*
Cronartium, *Cronartium ribicola,*
*Cryphonectriaceae,*
*Cryptobasidiaceae,*
Cryptocline, *Cryptocline cyclaminis,*
*Cryptomeliolo,*
Cryptosporella, *Cryptosporella umbrina,*
Cryptosporiopsis, *Cryptosporiopsis tarraconensis,*
Cryptosporium, *Cryptosporium minimum,*
Curvularia, *Curvularia lunata, Curvularia caricae-papayae, Curvularia penniseti, Curvularia senegalensis, Curvularia trifolii,*
*Cyclaneusma needle cast,*
Cylindrocarpon, *Cylindrocarpon ianthothele var. ianthothele, Cylindrocarpon magnusianum, Cylindrocarpon musae,*
Cylindrocladiella, *Cylindrocladiella camelliae, Cylindrocladiella parva,*
Cylindrocladium, *Cylindrocladium clavatum, Cylindrocladium lanceolatum, Cylindrocladium peruvianum, Cylindrocladium pteridis,*
Cylindrosporium, *Cylindrosporium cannabinum, Cylindrosporium juglandis, Cylindrosporium rubi,*
Cymadothea, *Cymadothea trifolii,*
Cytospora, *Cytospora palmarum, Cytospora personata, Cytospora sacchari, Cytospora sacculus, Cytospora terebinthi,*
Cytosporina, *Cytosporina ludibunda,*
Dactuliophora, *Dactuliophora elongata,*
Davidiella, *Davidiella dianthi, Davidiella tassiana,*
Deightoniella, *Deightoniella papuana, Deightoniella torulosa,*
Dendrophora, *Dendrophora marconii, Dendrophora erumpens,*
Denticularia, *Denticularia mangiferae,*
*Dermea pseudotsugae,*
*Diaporthaceae,*
Diaporthe, *Diaporthe arctii, Diaporthe dulcamarae, Diaporthe eres, Diaporthe helianthi, Diaporthe lagunensis, Diaporthe lokoyae, Diaporthe melonis, Diaporthe orthoceras, Diaporthe perniciosa, Diaporthe phaseolorum, Diaporthe phaseolorum var. caulivora, Diaporthe phaseolorum var. phaseolorum, Diaporthe phaseolorum var. soja, Diaporthe rudis, Diaporthe tanakae, Diaporthe toxica,*
Dicarpella, *Dicarpella dryina,*
Didymella, *Didymella applanata, Didymella bryoniae, Didymella fabae, Didymella lycopersici*
Didymosphaeria, *Didymosphaeria arachidicola, Didymosphaeria taiwanensis,*
Dilophospora, *Dilophospora alopecuri,*
Dimeriella, *Dimeriella sacchari,*
Diplocarpon, *Diplocarpon mespili, Diplocarpon rosae,*
Discosia, *Discosia artocreas,*
Discostroma, *Discostroma corticola,*
Distocercospora, *Distocercospora livistonae,*
Dothiorella, *Dothiorella brevicollis, Dothiorella dominicana, Dothiorella dulcispinae, Dothiorella gregaria,*
Drechslera, *Drechslera avenacea, Drechslera campanulata, Drechslera dematioidea, Drechslera gigantea, Drechslera glycines, Drechslera musae-sapientium, Drechslera teres f. maculata, Drechslera wirreganensis,*
Eballistra, *Eballistra lineata, Eballistra oryzae,*
*Eballistraceae,*
Echinodontium, *Echinodontium ryvardenii, Echinodontium tinctorium,*
*Ectendomeliola,*
Elsinoë, *Elsinoë ampelina, Elsinoë batatas, Elsinoë brasiliensis, Elsinoë leucospila, Elsinoë randii, Elsinoë rosarum, Elsinoë sacchari, Elsinoë theae, Elsinoë veneta,*
*Endomeliola,*
Endothia, *Endothia radicalis,*
Endothiella, *Endothiella gyrosa,*
*Entorrhizomycetes,*
Entyloma, *Entyloma ageratinae, Entyloma dahliae, Entyloma ellisii,*
Epicoccum, *Epicoccum nigrum,*
Eremothecium, *Eremothecium coryli, Eremothecium gossypii,*
*Erysiphales,*
Exobasidiaceae, *Exobasidium burtii, Exobasidium reticulatum, Exobasidium vaccinii var. japonicum, Exobasidium vaccinii-uliginosi, Exobasidium vexans,xxophiala alcalophila,*
Exophiala, *Exophiala angulospora, Exophiala attenuata, Exophiala calicioides, Exophiala castellanii, Exophiala dermatitidis, Exophiala dopicola, Exophiala exophialae, Exophiala heteromorpha, Exophiala hongkongensis, Exophiala jeanselmei, Exophiala lecanii-corni, Exophiala mansonii, Exophiala mesophila, Exophiala moniliae, Exophiala negronii, Exophiala phaeomuriformis, Exophiala pisciphila, Exophiala psychrophila, Exophiala salmonis, Exophiala spinifera,*
Fomes, *Fomes lamaënsis,*
Fomitopsis, *Fomitopsis rosea,*
Fusicladium *Fusicladium pisicola,*
Fusicoccum, *Fusicoccum aesculi, Fusicoccum amygdali, Fusicoccum quercus,*
Galactomyces, *Galactomyces candidum,*
Ganoderma, *Ganoderma brownii, Ganoderma lobatum, Ganoderma megaloma, Ganoderma meredithiae, Ganoderma orbiforme, Ganoderma philippii, Ganoderma sessile, Ganoderma tornatum, Ganoderma zonatum,*
Geastrumia, *Geastrumia polystigmatis,*
*Georgefischeriaceae,*
*Georgefischeriales,*
Geosmithia, *Geosmithia pallida,*
Geotrichum, *Geotrichum candidum, Geotrichum klebahnii,*
Gibberella, *Gibberella acuminata, Gibberella avenacea, Gibberella baccata, Gibberella cyanogena, Gibberella fujikuroi, Gibberella intricans, Gibberella pulicaris, Gibberella stilboides, Gibberella tricincta, Gibberella xylarioides, Gibberella zeae,*
Gibellina, *Gibellina cerealis,*
Gilbertella, *Gilbertella persicaria,*
*Gjaerumiaceae,*
Gliocladiopsis, *Gliocladiopsis tenuis,*
Gliocladium, *Gliocladium vermoeseni,*
Gloeocercospora, *Gloeocercospora sorghi,*
Gloeocystidiellum, *Gloeocystidiellum porosum,*
Gloeophyllum, *Gloeophyllum mexicanum, Gloeophyllum trabeum,*
Gloeoporus, *Gloeoporus dichrous,*
Gloeosporium, *Gloeosporium cattleyae, Gloeosporium theae-sinensis,*
Glomerella, *Glomerella cingulata, Glomerella graminicola, Glomerella tucumanensis,*
Gnomonia, *Gnomonia caryae, Gnomonia comari, Gnomonia dispora, Gnomonia iliau, Gnomonia rubi,*
Golovinomyces, *Golovinomyces cichoracearum,*
*Graphiola phoenicis,*
*Graphiolaceae,*
Graphium, *Graphium rigidum, Graphium rubrum,*
Graphyllium, *Graphyllium pentamerum,*
Grovesinia, *Grovesinia pyramidalis,*
Gymnoconia, *Gymnoconia nitens,*
Gymnopus, *Gymnopus dryophilus,*
Gymnosporangium, *Gymnosporangium kernianum, Gymnosporangium libocedri, Gymnosporangium nelsonii, Gymnosporangium yamadae,*
Haematonectria, *Haematonectria haematococco,*
Hansenula, *Hansenula subpelliculosa,*
Hapalosphaeria, *Hapalosphaeria deformans,*
Haplobasidion, *Haplobasidion musae,*
Helicobasidium, *Helicobasidium compactum, Helicobasidium longisporum, Helicobasidium purpureum,*
Helicoma, *Helicoma muelleri,*
Helminthosporium, *Helminthosporium cookei, Helminthosporium solani,*
Hendersonia, *Hendersonia creberrima, Hendersonia theicola,*
Hericium, *Hericium coralloides,*
Heterobasidion, *Heterobasidion irregulare, Heterobasidion occidentale,*
Hexagonia, *Hexagonia hydnoides,*
Hymenula, *Hymenula affinis,*
Hyphodermella, *Hyphodermella corrugata,*
Hyphodontia, *Hyphodontia aspera, Hyphodontia sambuci,*
Hypoxylon, *Hypoxylon tinctor,*
Inonotus, *Inonotus arizonicus, Inonotus cuticularis, Inonotus dryophilus, Inonotus hispidus, Inonotus ludovicianus,*
Irpex, *Irpex destruens, Irpex lacteus,*
Kabatiella, *Kabatiella caulivora,*
*Karnal bunt,*
*Koa wilt,*
Kretzschmaria, *Kretzschmaria zonata,*
Kuehneola, *Kuehneola uredinis,*
Kutilakesa, *Kutilakesa pironii,*
Laetiporus, *Laetiporus ailaoshanensis, Laetiporus baudonii, Laetiporus caribensis, Laetiporus conifericola, Laetiporus cremeiporus, Laetiporus gilbertsonii, Laetiporus huroniensis, Laetiporus montanus, Laetiporus portentosus, Laetiporus zonatus,*
Laxitextum, *Laxitextum bicolor,*
Leandria, *Leandria momordicae,*
Lentinus, *Lentinus tigrinus,*
Lenzites, *Lenzites betulina, Lenzites elegans,*
Leohumicola, *Leohumicola atra, Leohumicola incrustata, Leohumicola levissima,*
Leptodontidium, *Leptodontidium elatius,*
Leptographium, *Leptographium microsporum,*
Leptosphaerulina, *Leptosphaerulina crassiasca, Leptosphaerulina trifolii,*
Leptothyrium, *Leptothyrium nervisedum,*
Leptotrochila, *Leptotrochila medicaginis,*
Leucocytospora, *Leucocytospora leucostoma,*
Leucostoma, *Leucostoma auerswaldii, Leucostoma canker, Leucostoma kunzei, Leucostoma persoonii,*
Leveillula, *Leveillula compositarum, Leveillula leguminosarum, Leveillula tourica,*
Limacinula, *Limacinula tenuis,*
Linochora, *Linochora graminis,*
*Loose smut,*
Lopharia, *Lopharia crassa,*
Lophodermium, *Lophodermium aucupariae, Lophodermium schweinitzii,*
Macrophoma, *Macrophoma mangiferae, Macrophoma theicola,*
Macrosporium, *Macrosporium cocos,*
Magnaporthe, *Magnaporthe grisea, Magnaporthe salvinii,*
*Magnaporthiopsis,*
Mamianiella, *Mamianiella coryli,*
Marasmiellus, *Marasmiellus cocophilus, Marasmiellus stenophyllus,*
Marasmius, *Marasmius crinis-equi, Marasmius sacchari, Marasmius semiustus, Marasmius stenophyllus, Marasmius tenuissimus,*
Massarina, *Massarina walkeri,*
Mauginiella, *Mauginiella scaettae,*
Melampsora, *Melampsora lini, Melampsora occidentalis,*
Melanconis, *Melanconis carthusiana,*
Melanconium, *Melanconium juglandinum,*
Meliola, *Meliola mangiferae, Meliola zangii,*
Meruliopsis, *Meruliopsis ambigua,*
Microascus, *Microascus brevicaulis,*
Microbotryum, *Microbotryum silenes-dioicae, Microbotryum violaceum,*
Microsphaera, *Microsphaera coryli, Microsphaera diffusa, Microsphaera ellisii, Microsphaera euphorbiae, Microsphaera hommae, Microsphaera penicillata, Microsphaera vaccinii, Microsphaera verruculosa,*
Microstroma, *Microstroma juglandis,*
Moesziomyces, *Moesziomyces bullatus,*
Moniliophthora, *Moniliophthora roreri,*
Monilochaetes, *Monilochaetes infuscans,*
Monochaetia, *Monochaetia coryli, Monochaetia mali,*
Monographella, *Monographella albescens, Monographella cucumerina, Monographella nivalis,*
Monosporascus, *Monosporascus cannonballus, Monosporascus eutypoides,*
Monostichella, *Monostichella coryli,*
Mucor, *Mucor circinelloides, Mucor hiemalis, Mucor mucedo, Mucor paronychius, Mucor piriformis, Mucor racemosus,*
Mycena, *Mycena citricolor,*
Mycocentrospora, *Mycocentrospora acerina,*
Mycoleptodiscus, *Mycoleptodiscus terrestris,*
Didymella, *Didymella rabiei,*
*Mycosphaerella, Mycosphaerella recutita, Mycosphaerella rosicola, Mycosphaerella rubi, Mycosphaerella stigmina-platani, Mycosphaerella striatiformans,*
Mycovellosiella, *Mycovellosiella concors,*
Passalora, *Passalora fulva,*
Mycovellosiella, *Mycovellosiella koepkei, Mycovellosiella vaginae,*
Myriogenospora, *Myriogenospora aciculispora,*
Myrothecium, *Myrothecium roridum, Myrothecium verrucaria,*
Naevala, *Naevala perexigua,*
Naohidemyces, *Naohidemyces vaccinii,*
Nectria, *Nectria cinnabarina, Nectria ditissima, Nectria foliicola, Nectria mammoidea, Nectria mauritiicola, Nectria peziza, Nectria pseudotrichia, Nectria radicicola, Nectria ramulariae,*
Nectriella, *Nectriella pironii,*
Nemania, *Nemania diffusa, Nemania serpens,*
Neocosmospora, *Neocosmospora vasinfecta,*
Neodeightonia, *Neodeightonia phoenicum,*
Neoerysiphe, *Neoerysiphe galeopsidis,*
Neofabraea, *Neofabraea perennans,*
Neofusicoccum, *Neofusicoccum mangiferae,*
Oidiopsis, *Oidiopsis gossypii,*
Oidium, *Oidium arachidis, Oidium caricae-papayae, Oidium indicum, Oidium mangiferae, Oidium manihotis,*
Olpidium, *Olpidium brassicae,*
Omphalia, *Omphalia tralucida,*
Ophiobolus, *Ophiobolus anguillides, Ophiobolus cannabinus,*
*Ophioirenina,*
Ovulinia, *Ovulinia azaleae,*
Oxyporus, *Oxyporus corticola,*
Ozonium, *Ozonium texanum,*
Peltaster, *Peltaster fructicola,*
Penicillium, *Penicillium expansum, Penicillium funiculosum,*
*Peniophora,*
Periconia, *Periconia circinata,*
Periconiella, *Periconiella cocoes,*
Peridermium, *Peridermium californicum,*
Pestalosphaeria, *Pestalosphaeria concentrica,*
Pestalotia, *Pestalotia longiseta, Pestalotia rhododendri,*
Pestalotiopsis, *Pestalotiopsis adusta, Pestalotiopsis arachidis, Pestalotiopsis disseminata, Pestalotiopsis guepini, Pestalotiopsis leprogena, Pestalotiopsis longiseta, Pestalotiopsis mangiferae, Pestalotiopsis palmarum, Pestalotiopsis sydowiana, Pestalotiopsis theae,*
Peyronellaea, *Peyronellaea curtisii,*
Phacidiopycnis, *Phacidiopycnis padwickii,*
Phaeochoropsis, *Phaeochoropsis mucosa,*
Phaeocytostroma, *Phaeocytostroma iliau, Phaeocytostroma sacchari,*
Phaeoisariopsis, *Phaeoisariopsis bataticola,*
Phaeoramularia, *Phaeoramularia heterospora, Phaeoramularia indica, Phaeoramularia manihotis,*
Phaeoseptoria, *Phaeoseptoria musae,*
Phaeosphaerella, *Phaeosphaerella mangiferae, Phaeosphaerella theae,*
Phaeosphaeria, *Phaeosphaeria avenaria, Phaeosphaeria herpotrichoides, Phaeosphaeria microscopica, Phaeosphaeria nodorum,*
Phaeosphaeriopsis, *Phaeosphaeriopsis obtusispora,*
Phaeotrichoconis, *Phaeotrichoconis crotalariae,*
Phialophora, *Phialophora asteris, Phialophora cinerescens, Phialophora gregata, Phialophora tracheiphila,*
Phoma, *Phoma clematidina, Phoma costaricensis, Phoma cucurbitacearum, Phoma destructiva, Phoma draconis, Phoma exigua, Phoma exigua, Phoma exigua var. foveata, Phoma exigua, Phoma glomerata, Phoma glycinicola, Phoma herbarum, Phoma insidiosa, Phoma medicaginis, Phoma microspora, Phoma narcissi, Phoma nebulosa, Phoma oncidii-sphacelati, Phoma pinodella, Phoma sclerotioides, Phoma strasseri,*
Phomopsis, *Phomopsis asparagi, Phomopsis asparagicola, Phomopsis cannabina, Phomopsis coffeae, Phomopsis ganjae, Phomopsis javanica, Phomopsis longicolla, Phomopsis mangiferae, Phomopsis prunorum, Phomopsis sclerotioides, Phomopsis theae,*
Phragmidium, *Phragmidium mucronatum, Phragmidium rosae-pimpinellifoliae, Phragmidium rubiidaei, Phragmidium violaceum,*
Phyllachora, *Phyllachora banksiae, Phyllachora cannabis, Phyllachora graminis, Phyllachora gratissima, Phyllachora musicola, Phyllachora pomigena, Phyllachora sacchari,*
Phyllactinia,
Phyllosticta, *Phyllosticta alliariaefoliae Phyllosticta arachidis-hypogaeae, Phyllosticta batatas, Phyllosticta capitalensis, Phyllosticta carpogena, Phyllosticta coffeicola, Phyllosticta concentrica, Phyllosticta coryli, Phyllosticta cucurbitacearum, Phyllosticta cyclaminella, Phyllosticta erratica, Phyllosticta hawaiiensis, Phyllosticta lentisci, Phyllosticta manihotis, Phyllosticta micropuncta, Phyllosticta mortonii, Phyllosticta nicotianae, Phyllosticta palmetto, Phyllosticta penicillariae, Phyllosticta perseae, Phyllosticta pseudocapsici, Phyllosticta sojaecola, Phyllosticta theae, Phyllosticta theicola,*
Phymatotrichopsis, *Phymatotrichopsis omnivora,*
Physalospora, *Physalospora disrupta, Physalospora perseae,*
Physoderma, *Physoderma alfalfae, Physoderma leproides, Physoderma trifolii,*
Physopella, *Physopella ampelopsidis,*
Pileolaria, *Pileolaria terebinthi,*
Piricaudiopsis, *Piricaudiopsis punicae, Piricaudiopsis rhaphidophorae, Piricaudiopsis rosae,*
Plenodomus, *Plenodomus destruens, Plenodomus meliloti,*
Pleosphaerulina, *Pleosphaerulina sojicola,*
Pleospora, *Pleospora alfalfae, Pleospora betae, Pleospora herbarum, Pleospora lycopersici, Pleospora tarda, Pleospora theae,*
*Pleuroceras,*
Podosphaera, *Podosphaera fuliginea, Podosphaera fusca, Podosphaera leucotricha, Podosphaera macularis, Podosphaera pannosa,*
Polyscytalum, *Polyscytalum pustulans,*
Poria, *Poria hypobrunnea,*
Postia, *Postia tephroleuca,*
*Powdery mildew,*
Pseudocercospora, *Pseudocercospora arecacearum, Pseudocercospora cannabina, Pseudocercospora fuligena, Pseudocercosporella herpotrichoides, Pseudocercospora gunnerae, Pseudocercospora pandoreae, Pseudocercospora puderi, Pseudocercospora rhapisicola, Pseudocercospora theae, Pseudocercospora vitis, Pseudocercosporella capsellae,*
Pseudocochliobolus, *Pseudocochliobolus eragrostidis,*
Pseudoepicoccum, *Pseudoepicoccum cocos,*
Pseudopeziza, *Pseudopeziza jonesii, Pseudopeziza medicaginis, Pseudopeziza trifolii,*
Pseudoseptoria, *Pseudoseptoria donacis,*
*Pucciniaceae,*
Pucciniastrum, *Pucciniastrum americanum, Pucciniastrum arcticum, Pucciniastrum epilobii, Pucciniastrum hydrangeae,*
Pycnostysanus, *Pycnostysanus azaleae,*
Pyrenochaeta, *Pyrenochaeta lycopersici, Pyrenochaeta terrestris,*
Pyrenopeziza, *Pyrenopeziza brassicae,*
Ramichloridium, *Ramichloridium musae,*
Ramulispora, *Ramulispora sorghi, Ramulispora sorghicola,*
Rhinocladium, *Rhinocladium corticola,*
Rhizophydium, *Rhizophydium graminis,*
Rhizopus, *Rhizopus arrhizus, Rhizopus circinans, Rhizopus microsporus, Rhizopus oryzae,*
Rhytisma, *Rhytisma punctatum, Rhytisma vitis,*
Rigidoporus, *Rigidoporus vinctus,*
Rosellinia, *Rosellinia arcuata, Rosellinia bunodes, Rosellinia necatrix, Rosellinia pepo,*
Saccharicola, *Saccharicola taiwanensis,*
Schiffnerula, *Schiffnerula cannabis,*
Schizophyllum, *Schizophyllum commune,*
Schizopora, *Schizopora flavipora,*
Schizothyrium, *Schizothyrium pomi,*
Sclerophthora, *Sclerophthora macrospora,*
Sclerotium, *Sclerotium cinnamomi, Sclerotium delphinii,*
Scytinostroma, *Scytinostroma galactinum,*
Seimatosporium, *Seimatosporium mariae, Seimatosporium rhododendri,*
Selenophoma, *Selenophoma linicola,*
Septobasidium, *Septobasidium bogoriense, Septobasidium euryae-groffii, Septobasidium gaoligongense, Septobasidium pilosum, Septobasidium polygoni, Septobasidium pseudopedicellatum, Septobasidium theae,*
Septocyta, *Septocyta ruborum,*
Serpula, *Serpula lacrymans,*
Setosphaeria, *Setosphaeria rostrata, Setosphaeria turcica,*
Spencermartinsia, *Spencermartinsia pretoriensis,*
Sphaceloma, *Sphaceloma arachidis, Sphaceloma menthae, Sphaceloma perseae, Sphaceloma poinsettiae, Sphaceloma sacchari, Sphaceloma theae,*
Sphacelotheca, *Sphacelotheca reiliana, Sphaerotheca castagnei,*
Sphaerulina, *Sphaerulina oryzina, Sphaerulina rehmiana, Sphaerulina rubi,*
Sphenospora, *Sphenospora kevorkianii,*
Spilocaea, *Spilocaea oleaginea,*
Sporisorium, *Sporisorium cruentum, Sporisorium ehrenbergii, Sporisorium scitamineum, Sporisorium sorghi,*
Sporonema, *Sporonema phacidioides,*
Stagonospora, *Stagonospora avenae, Stagonospora meliloti, Stagonospora recedens, Stagonospora sacchari, Stagonospora tainanensis,*
*Stagonosporopsis,*
Stegocintractia, *Stegocintractia junci,*
Stemphylium, *Stemphylium alfalfae, Stemphylium bolickii, Stemphylium cannabinum, Stemphylium globuliferum, Stemphylium lycopersici, Stemphylium sarciniforme, Stemphylium solani, Stemphylium vesicarium,*
Stenella, *Stenella anthuriicola,*
*Stigmatomycosis,*
Stigmina, *Stigmina carpophila, Stigmina palmivora, Stigmina platani-racemosae,*
Stromatinia, *Stromatinia cepivora,*
Sydowiella, *Sydowiella depressula,*
*Sydowiellaceae,*
Synchytrium, *Synchytrium endobioticum,*
Tapesia, *Tapesia acuformis, Tapesia yallundae,*
Taphrina, *Taphrina coryli, Taphrina potentillae,*
Thanatephorus, *Thanatephorus cucumeris,*
Thecaphora, *Thecaphora solani,*
Thielaviopsis, *Thielaviopsis basicola, Thielaviopsis ceramica,*
Thyrostroma, *Thyrostroma compactum,*
Tiarosporella, *Tiarosporella urbis-rosarum,*
Tilletia, *Tilletia barclayana, Tilletia caries, Tilletia controversa, Tilletia laevis, Tilletia tritici, Tilletia walkeri,*
*Tilletiariaceae,*
*Togniniaceae,*
Tranzschelia, *Tranzschelia pruni-spinosae,*
Trichoderma, *Trichoderma koningii, Trichoderma paucisporum, Trichoderma songyi, Trichoderma theobromicola, Trichoderma viride,*
Tubercularia, *Tubercularia lateritia,*
Tunstallia, *Tunstallia aculeata,*
Typhula, *Typhula blight, Typhula idahoensis, Typhula incarnata, Typhula ishikariensis, Typhula variabilis,*
Ulocladium, *Ulocladium consortiale,*
*Uncinula,*
Uredo, *Uredo behnickiana, Uredo kriegeriana, Uredo musae, Uredo nigropuncta, Uredo rangelii,*
Urocystis, *Urocystis agropyri, Urocystis brassicae, Urocystis occulta,*
Uromyces, *Uromyces apiosporus, Uromyces appendiculatus, Uromyces beticola, Uromyces cicerisarietini, Uromyces dianthi, Uromyces euphorbiae, Uromyces graminis, Uromyces inconspicuus, Uromyces lineolatus, Uromyces musae, Uromyces oblongus, Uromyces pisi-sativi, Uromyces proëminens, Uromyces medicaginis, Uromyces trifolii-repentis, Uromyces viciae-fabae,*
Urophlyctis, *Urophlyctis leproides, Urophlyctis trifolii,*
*Ustilaginales,*
Ustilago, *Ustilago avenae, Ustilago esculenta, Ustilago hordei, Ustilago maydis, Ustilago nigra, Ustilago nuda, Ustilago scitaminea, Ustilago tritici,*
Vankya, *Vankya ornithogali,*
*Velvet blight,*
Veronaea, *Veronaea musae,*
Verticillium, *Verticillium albo-atrum, Verticillium alfalfae, Verticillium dahliae, Verticillium isaacii, Verticillium klebahnii, Verticillium longisporum, Verticillium nonalfalfae, Verticillium theobromae, Verticillium wilt, Verticillium zaregamsianum,*
Waitea, *Waitea circinata,*
*Westea,*
*Wheat leaf rust,*
*Wheat mildew,*
Wuestneiopsis, *Wuestneiopsis georgiana,*
Xeromphalina, *Xeromphalina fraxinophila,*
Zopfia, *Zopfia rhizophila,*
Zygosaccharomyces, *Zygosaccharomyces bailii, Zygosaccharomyces florentinus,*
*Zythiostroma.*

De préférence, les couples champignons, oomycètes ou bactéries vs. plantes de culture visés par l'invention sont les suivants :
**Blé *(Triticum sativum)***
   *Claviceps purpurea, Erysiphe graminis, Fusarium avenaceum, Fusarium culmorum, Fusarium graminearum, Fusarium langsethiae, Fusarium poae, Fusarium pseudograminearum, Gaeumannomyces graminis, Leptosphaeria nodorum, Microdochium spp., Mycosphaerella graminicola, Oculimacula acuformis, Oculimacula yallundae, Puccinia recôndita, Puccinia striiformis, Pyrenophora tritici-repentis, Rhizoctonia cerealis, Microdochium* et *Zymoseptoria tritici*
**Mais (*Zea mays*)**
   *Fusarium graminearum, Fusarium proliferatum, Fusarium subglutinans, Fusarium verticillioides*
**Orge *(Hordeum vulgare)***
   *Blumeria graminis, Fusarium spp, Pyrenophora teres, Ramularia collo-cygni, Rhynchosporium secalis*
**Riz (Oryza sativa)**
   *Cochliobolus miyabeanus, Fusarium fijikuroï, Magnaporthe oryzae, Microdochium oryzae, Pyricularia oryzae, Rhizoctonia oryzae, Rhizoctonia solani, Sarocladium oryzae, Ustilaginoides virens*
**Pomme de terre (*Solanum tuberosum*)**
   *Alternia alternata, Alternaria solani, Phytophtora infestans, Rhizoctonia solani*
**Vigne (*Vinis vitifera*)**
   *Botrytis cinerea, Erysiphe necator, Plasmopara viticola, Guignardia bidwelli, Erisyphe necator, Diplodia seriata*
**Soja (*Glycine max*)**
   *Cercopora kikuchii, Colletotrichum dematium, Corynespora cassiicola , Fusarium graminearum, Pythium spp., Rhizoctonia solani, Sclerotinia sclerotiorum , Septoria glycines*
**Pommier (*Malus domestica*)**
   *Monilia fructigena, Monilia laxa, Pezzicula alba, Pezzicula malicorticis, Venturia inaequalis*
**Tomate (*Lycopersicon esculentum*)**
   *Phytophtora infestans*
**Haricot (*Phaseolus vulgaris*)**
   Uromyces appendiculatus
**Radis *(Raphanus sativus)***
   *Alternaria brassicae*
**Tous les Fruits et légumes**
   *Botrytis cinerea*
Fraisier ***(Fragaria** sp)*
   *Colletotrichum acutatum*
**Carotte *(Daucus carota)***
   *Aternaria alternata, Alternaria douci, Alternaria radicina*
**Pêche *(Prunus persica)* et abricot *(Prunus armeniaca)***
   *Monilia fructicola, Monilia fructigena, Monilia laxa*

De manière particulièrement préférée, les couples champignons ou bactéries vs. plantes de culture visés par l'invention sont les suivants :
*Blé : Fusarium graminearum, Microdochium* et *Zymoseptoria tritici*
**Vigne:** *Botrytis cinérea, Erysiphe necator, Plasmopara viticola, Guignardia bidwelli, Erisyphe necator, Diplodia seriata*
**Pomme de terre** *: Alternia alternata, Alternaria solani, Phytophtora infestans, Rhizoctonia solani*
**Tomate:** *Phytophtora infestans*

### Procédé de lutte

L'invention concerne un procédé de lutte contre les champignons et/ou les oomycètes des plantes et semence de culture caractérisé par les étapes suivantes :
- Récolte de cellules fraiches d'une ou plusieurs micro-algue(s) du genre *Amphidinium ;*
- Congélation et/ou lyophilisation desdites cellules ;
- Suspension dudit lyophilisat ou desdites cellules congelées dans de l'eau, dans un rapport en poids de 1:200 à 1:2 à une température supérieure à 60°C ;
- Application sur des plantes de culture et/ou enrobage de semences de l'extrait ainsi obtenu. Cette lutte peut être curative ou préventive, de préférence curative.

De préférence, la mise en suspension desdites cellules congelées ou dudit lyophilisat dans un solvant inorganique ou organique est réalisée dans un rapport en poids lyophilisat ou biomasse/solvant compris entre 1:100 et 1:50.

De préférence, la mise en suspension desdites cellules congelées ou dudit lyophilisat dans l'eau est réalisée à une température supérieure à 80°C, de manière particulièrement préférée à une température supérieure à 90°C. De préférence, la mise en suspension desdites cellules congelées ou dudit lyophilisat dans l'eau dure moins de 5 minutes, de préférence moins de 3 minutes, de préférence moins de 1 minute. De préférence, la température est ensuite ramenée rapidement à température ambiante. De préférence, la température du mélange est ramenée proche de la température ambiante en plaçant le mélange dans un environnement froid, par exemple à une température proche de 0°C, ou en ajoutant au mélange un solvant inorganique à une température proche de 0°C.

La suspension est réalisée soit en ajoutant aux dites cellules congelées ou dudit lyophilisat l'eau portée préalablement à la température désirée, soit l'eau est ajoutée et le mélange resuspendu est ajusté à la température désirée.

Ramener « rapidement » la température proche de la température ambiante signifie en moins de 5 minutes, de préférence en moins de 3 minutes, de préférence en moins de 1 minute.

Les cellules fraiches récoltées et extraites proviennent d'une culture cellulaire dans des conditions de température, photopériode et salinité adaptées à la souche concernée jusqu'à une concentration cellulaire comprise entre 5.10⁴ cellules/ml et 5.10⁶ cellules/ml, préferentiellement une concentration cellulaire comprise entre 5.10⁵ cellules/ml et 1.10⁶ cellules/ml.

Les cellules sont cultivées pendant 5 à 20 jours.

L'intensité lumineuse est comprise entre 40 µE et 200 µE, de préférence comprise entre 70 µE et 100 µE.

La température de culture est généralement comprise entre 17°C et 25°C.

La photopériode jour/nuit est de préférence comprise entre 8h/16h et 16h/8h.

La salinité minimum est de 15 ppt.

Selon un mode de réalisation particulier, *Amphidinium carterae* est cultivé comme suit : les cellules sont incubées dans du milieu d'eau de mer naturel ou artificiel à une température comprise entre 17 à 25°C avec un cycle jour/nuit compris entre 8h/16h et 16h/8h, de préférence de 16h/8h.

L'application sur les plantes de culture peut être réalisée par tout moyen connu de l'homme du métier permettant d'atteindre les parties de plantes touchées par le champignon.

L'extrait est appliqué à une dose comprise entre 0,005 g/L et 20 g/L, de préférence entre 0,5 g/L et 10 g/L, de manière particulièrement préférée entre 1 g/L et 5 g/L.

L'enrobage des semences peut être réalisé par toute technique connue de l'homme du métier qui permet de maintenir l'actif en contact avec la semence.

Par exemple, l'enrobage peut être réalisé par poudrage ou par pulvérisation.

Par exemple, l'enrobage peut comprendre des formulants et des adjuvants.

Les formulants ont pour objectif de rendre possible l'application et la tenue de la ou des substances actives sur le grain, en proportion égale et constante pendant tout le procédé d'application du produit et ceci à des doses très faibles.

Les formulants comprennent : des solvants organiques ou de l'eau, des dispersants, des émulgateurs, des tensioactifs ou des mouillants, des colorants...

Les tensio-actifs et les émulgateurs ont la propriété de réunir et de maintenir ensemble de façon stable deux liquides incompatibles.

Différents adjuvants peuvent être appliqués sur la semence. Les pelliculants correspondent à l'application d'un film microporeux à la surface de la semence. Ils ne modifient ni la forme ni la taille de la semence. Ils améliorent la couverture et l'homogénéité du traitement. Lors de l'utilisation des semences par l'agriculteur, ils améliorent le confort de l'utilisateur au moment du semis en supprimant les poussières et en facilitant l'écoulement des semences dans le semoir. Ils améliorent l'action de la ou des substances actives en condition de culture. Les enrobants modifient la forme, la taille et le poids de la semence. Ils améliorent la précision du semis.

Le procédé de lutte selon l'invention est particulièrement approprié contre une Fusariose, de préférence une Fusariose citée dans le Tableau 1.

**Tableau 1 : récapitulatif des fusarioses**

| **Nom de la maladie** | **Agent pathogène** | **code OEPP** |
|---|---|---|
| fusariose basale de l'asperge | *Fusarium culmorum* | FUSACU |
| fusariose basale du haricot | *Fusarium solani f. sp. phaseoli* | FUSASH |
| fusariose basale du pois | *Fusarium solani f. sp. pisi* | FUSASI |
| fusariose de la betterave | *Fusarium oxysporum f. sp. betae* | FUSABE |
| fusariose de la pomme de terre | *Fusarium coeruleum* | FUSASC |
| fusariose de la reine-marguerite | *Fusarium oxysporum f. sp. conglutinans* | FUSACO |
| fusariose de la tige du mais | *Gibberella fujikuroi* | GIBBFU |
| fusariose de la tige du maïs | *Fusarium culmorum* | FUSACU |
| fusariose de la tige du maïs | *Gibberella zeae* | GIBBZE |
| fusariose de la vanille | *Fusarium oxysporum f. sp. vanillae* | FUSAVN |
| fusariose de l'ananas | *Gibberella fujikuroi var. subglutinans* | GIBBFS |
| fusariose de l'épi du maïs | *Fusarium poae* | FUSAPO |
| fusariose de l'épi du maïs | *Fusarium tricinctum* | FUSATI |
| fusariose de l'œillet | *Fusarium oxysporum f. sp. dianthi* | FUSADI |
| fusariose des broméliacées | *Fusarium oxysporum f. sp. aechmeae* | FUSAAE |
| fusariose des bulbes | *Fusarium oxysporum f. sp. gladioli* | FUSAGL |
| fusariose des céréales | *Fusarium culmorum* | FUSACU |
| fusariose des céréales | *Gibberella rosea* | FUSARO |
| fusariose des céréales | *Gibberella avenacea* | GIBBAV |
| fusariose des céréales | *Gibberella intricans* | GIBBIN |
| fusariose des céréales | *Monographella nivalis* | MONGNI |
| fusariose des épis | *Gibberella zeae* | GIBBZE |
| fusariose des racines de l'asperge | *Fusarium oxysporum f. sp. asparagi* | FUSAAS |
| fusariose des racines des cactées | *Fusarium oxysporum f. sp. opuntiarum* | FUSAOP |
| fusariose des racines et du collet de la tomate | *Fusarium oxysporum f. sp. radicis-lycopersici* | FUSARL |
| fusariose des racines et du collet du concombre | *Fusarium oxysporum f. sp. cucumerinum* | FUSACC |
| fusariose du blé | *Gibberella fujikuroi* | GIBBFU |
| fusariose du cacaoyer | *Albonectria rigidiuscula* | CALORI |
| fusariose du caféier | *Gibberella stilboides* | GIBBST |
| fusariose du carthame | *Fusarium oxysporum f. sp. carthami* | FUSACA |
| fusariose du cognassier | *Gibberella baccata* | GIBBBA |
| fusariose du collet des cucurbitacées | *Fusarium solani f. sp. cucurbitae* | FUSASU |
| fusariose du cotonnier | *Fusarium oxysporum f. sp. vasinfectum* | FUSAVA |
| fusariose du gerbéra | *Fusarium oxysporum f. sp. gerberae* | FUSAGE |
| fusariose du glaïeul | *Fusarium oxysporum f. sp. gladioli* | FUSAGL |
| fusariose du lin | *Fusarium oxysporum f. sp. lini* | FUSALI |
| fusariose du maïs | *Gibberella acuminata* | GIBBAC |
| fusariose du maïs | *Gibberella fujikuroi var. subglutinans* | GIBBFS |
| fusariose du maïs | *Gibberella zeae* | GIBBZE |
| fusariose du palmier à huile | *Fusarium oxysporum f. sp. elaeidis* | FUSAEL |
| fusariose du soja | *Fusarium oxysporum f. sp. glycines* | FUSAGY |
| fusariose du soja | *Fusarium oxysporum f. sp. tracheiphilum* | FUSATR |
| fusariose du tubercule de la pomme de terre | *Gibberella cyanogena* | GIBBCN |
| fusariose moniliforme | *Gibberella fujikuroi* | GIBBFU |
| fusariose nivale | *Monographella nivalis* | MONGNI |
| fusariose roseum | *Gibberella rosea* | FUSARO |
| fusariose vasculaire | *Fusarium oxysporum* | FUSAOX |
| fusariose vasculaire de la lentille | *Fusarium oxysporum f. sp. lentis* | FUSALE |
| fusariose vasculaire de la pastèque | *Fusarium oxysporum f. sp. niveum* | FUSANV |
| fusariose vasculaire de la tomate | *Fusarium oxysporum f. sp. lycopersici* | FUSALY |
| fusariose vasculaire de la tulipe | *Fusarium oxysporum f. sp. tulipae* | FUSATU |
| fusariose vasculaire des crucifères | *Fusarium oxysporum f. sp. conglutinans* | FUSACO |
| fusariose vasculaire du caféier | *Gibberella xylarioides* | GIBBXY |
| fusariose vasculaire du chou | *Fusarium oxysporum f. sp. conglutinans* | FUSACO |
| fusariose vasculaire du chrysanthème | *Fusarium oxysporum f. sp. chrysanthemi* | FUSACH |
| fusariose vasculaire du concombre | *Fusarium oxysporum f. sp. cucumerinum* | FUSACC |
| fusariose vasculaire du cyclamen | *Fusarium oxysporum var. aurantiacum* | FUSAAU |
| fusariose vasculaire du fraisier | *Fusarium oxysporum f. sp. fragariae* | FUSAFR |
| fusariose vasculaire du haricot | *Fusarium oxysporum f. sp. phaseoli* | FUSAPH |
| fusariose vasculaire du melon | *Fusarium oxysporum f. sp. melonis* | FUSAME |
| fusariose vasculaire du pois | *Fusarium oxysporum f. sp. pisi* | FUSAPI |
| fusariose vasculaire du pois chiche | *Gibberella baccata* | GIBBBA |
| fusariose vasculaire du pois-chiche | *Fusarium oxysporum f. sp. ciceris* | FUSACI |
| fusariose vasculaire du radis | *Fusarium oxysporum f. sp. raphani* | FUSARA |

Le procédé de lutte selon l'invention est particulièrement approprié pour les couples champignons vs. plantes de culture suivants :
***Blé :** Fusarium graminearum, Microdochium nivale* et *Zymoseptoria tritici*
**Vigne:** *Botrytis cinerea, Plasmopara viticola, Guignardia bidwelli, Erisyphe necator, Diplodia seriata*
**Pommier :** *Venturia inaequalis*
**Bananier :** *Fusarium oxysporum* et *Mycosphaerella fijiensis*

### EXEMPLES

### MATERIEL & METHODES

### Exemple 1 : Culture des micro-algues

La micro-algue *Amphidinium carterae,* souche AC208, provient de Algobank (Caen) et les micro-algues *Prymnesium parvum,* souche RCC 1436, et *Phaeodactylum tricornutum,* souche CCMP 632, proviennent de la banque de micro-organismes marins de Roscoff (RCC : Roscoff Culture Collection). Ces micro-algues sont cultivées dans de l'eau de mer artificielle L1 (https://ncma.bigelow.org/algal-recipes) à 19°C avec un cycle jour/nuit de 12H/12H. L'intensité lumineuse utilisée est de 100 µE. La biomasse est récupérée en fin de phase exponentielle de croissance par centrifugation (15 min à 3000 RPM). Le culot cellulaire obtenu est congelé puis soumis à la lyophilisation en utilisant un lyophilisateur de laboratoire (Alpha 1-2 LDplus, labconco) afin de conserver de manière stable la matière active sur une longue durée. Après lyophilisation, la matière sèche est pesée.

### Exemple 2 : Préparation de l'extrait

Afin d'extraire la matière active de la matière sèche de l'exemple 1, 20 mg de matière sèche sont resuspendus dans 1 mL d'eau distillée à 100°C. Après incubation pendant 2 minutes à température ambiante (20-25°C) l'extrait est conservé dans la glace puis centrifugé 5 min à 10 000 RPM à température ambiante. Le surnageant contenant la matière active est congelé dans de l'azote liquide afin de conserver ses propriétés anti-fongiques sur longue durée.

### Exemple 3 : Test de germination de Fusarium graminearum

Les spores de *Fusarium* graminearum sont cultivées dans le milieu appauvri « Mung bean ». Les spores sont séparées du mycélium par filtration sur du miracloth (Calbiochem), centrifugées puis resuspendues à 1,6.10⁶ spores/mL. Environ 16 000 spores sont incubées en présence de la solution contrôle ou de l'extrait d'A. *carterae* à différentes concentrations. Après une incubation de 10 min à température ambiante, les spores sont disposées sur une lamelle pour un dénombrement de la germination après 6H ou sur une boite de pétri pour une observation de la croissance du mycélium après 72H.

### ESSAIS

Des extraits de différentes espèces de microorganismes marins appartenant à trois *phylum* majeurs du phytoplancton, les dinoflagellés, les haptophytes et les diatomées, ont été testés pour une potentielle activité anti-fongique sur des champignons cryptogames. Ces micro-algues ont la capacité de produire des toxines leur permettant de fortement proliférer en rentrant en compétition avec d'autres espèces et donc sont des sources potentielles de molécules pouvant exhiber une activité anti-fongique. Les extraits de chaque micro-algue ont été obtenus selon l'Exemple 2. Pour tester l'effet de ces extraits sur la survie de champignons phytopathogènes, les extraits lyophilisés sont resuspendus dans de l'eau puis mis en contact avec une quantité donnée de spores de *Fusarium graminearum.*

### Exemple 4 : Inhibition de la croissance et de la germination des spores Fusarium graminearum

La capacité de *F. graminearum* de former du mycélium sur du milieu gélosé en présence de ces extraits est testée 72H après. Sur les trois extraits testés (*Prymnesium parvum, Amphidinium carterae* et *Phaeodactylum tricornutum*), seul l'extrait *d'Amphidinium carterae* selon l'Exemple 2 a un effet inhibiteur de la formation de mycélium (Figure 1A).

Pour confirmer ce résultat, un test de dose-réponse a été effectué en incubant les spores avec l'extrait d'*A. carterae* selon l'Exemple 2 dilué à des concentrations différentes (Figure 1B). Il s'avère que cet extrait a une activité fongique qui est dose dépendante avec une concentration minimale pour obtenir 100% d'inhibition (CMI) de 0,4 g/L (Figure 1B).

Enfin, l'extrait d'A. *carterae* selon l'Exemple 2 a été testé pour inhiber la germination des spores de *F. graminearum.* Les résultats *in vitro* obtenus 6 heures après incubation avec l'extrait démontre une inhibition totale de la germination des spores à une concentration de 2 g/L (Figure 1C) suggérant que l'extrait inhibe la germination des spores ainsi que la croissance du mycélium de *F. graminearum.*

### Exemple 5 : Influence de la lyophilisation de l'extrait d'A. carterae sur l'activité anti-fongique de l'extrait

Les inventeurs ont déterminé si la lyophilisation de l'extrait d'A. *carterae* inhibait ou non l'activité anti-fongique. Pour cela, une culture d'A. *carterae* a été extraite selon l'Exemple 2 puis la moitié de l'extrait a été congelée à -80°C alors que l'autre moitié de l'extrait a été lyophilisée puis resuspendue dans de l'eau distillée. Ces extraits ont été testés à différentes concentrations pour leur capacité à inhiber la croissance de *F. graminearum* selon l'Exemple 3. Les résultats démontrent que dans les deux cas, extrait congelé ou extrait lyophilisé, une inbition complète de la croissance de *F. graminearum* est obtenue à une concentration de 1 g/L et perdure jusqu'à 5 g/L (Figure 1D). En conclusion, la lyophilisation de l'extrait d'A. *carterae* n'affecte en aucun cas son activité anti-fongique.

### Exemple 6 : Tests sur des plants de blé infectés en conditions contrôlées

Des épis de blé ont été inoculés avec des spores de *F. graminearum* en conditions contrôlées puis 24H après l'extrait d'A. *carterae* de l'Exemple 2 a été appliqué La lecture des symptômes s'est faite à 20 jours (400°J) et à 22 jours (450°j) après infection (Figure 2A). Le nombre d'épis présentant des symptômes (l'incidence de la maladie) ainsi que la note des symptômes (la sévérité de la maladie, note de 0 à 9) ont été rapportés dans les deux cas : en absence (témoin) et en présence de l'extrait de l'Exemple 2. En comparaison avec le traitement témoin, la présence de l'extrait permet de réduire significativement, de l'ordre de 30%, le nombre d'épis attaqué par la maladie. De plus, ces épis présentent des symptômes ayant une sévérité réduite d'environ 50% (Figure 2B). Ces résultats démontrent que l'extrait d'A. *carterae* de l'Exemple 2 possède une activité anti-fongique significative sur la croissance de champignons phytopathogènes affectant le blé en conditions *in vitro* et *in planta.*

### Exemple 7 : Tests sur plusieurs familles de phytopathogènes de la vigne : Botrytis cinerea, un ascomycète responsable de la pourriture grise, Plasmopara viticola, un oomycète responsable du mildiou, Erisyphe necator, un ascomycète responsable de l'oïdium et Diplodia seriata, un des agents causals de l'esca, la maladie du bois.

A une concentration de 1 g/L, l'extrait d'A. *carterae* de l'exemple 2 inhibe totalement la croissance de *P. viticola* sur feuille détachée (Figure 3A, partie gauche), alors que dans les mêmes conditions l'extrait n'a pas d'effet sur la croissance *d'E. necator* (Figure 3A, partie droite). Des tests *in vitro* ont été menés sur *Botrytis cinerea* indiquant que l'extrait inhibe totalement la croissance de *B. cinerea à* une concentration de 5 g/L (Figure 3B). L'activité anti-fongique de l'extrait sur *B. cinerea* a été confirmée sur feuille détachée de vigne (Figure 3B). Par ailleurs, des tests ont été effectués *in vitro* sur les différentes familles de champignons responsables de l'esca de la vigne. Les résultats démontrent que l'extrait à une concentration de 2 g/L inhibe de manière importante la croissance de ces champignons (Figure 3C).

### Exemple 8 : Tests in vitro sur Microdochium majus, Fusarium graminearum, Zymoseptoria tritici, Fusarium oxysporum, Rhizoctonia solani et Phytophthora infestans

Afin de déterminer si l'extrait d'A. *carterae* selon l'Exemple 2 présente une activité anti-fongique sur un spectre large de champignons phytopathogènes, des tests ont été effectués sur d'autres champignons responsables de la maladie fusariose : *Fusarium oxysporum* (fusariose du bananier) et *Microdochium* (fusariose du blé) ainsi que sur une autre maladie importante du blé : la septoriose causée par *Zymoseptoria tritici.* Des tests ont aussi été effectués sur deux agents pathogènes de la pomme de terre : le basidiomycète *Rhizoctonia solani* et l'oomycete *Phytophthora infestans.* Dans tous les cas, l'extrait d'A. *carterae* selon l'Exemple 2 limitait fortement la croissance des champignons et de l'oomycète en condition *in vitro* (Tableaux 2 à 6).

**Tableau 2. Efficacité de l'extrait de micro-algue in vitro à l'encontre de macroconidies de F. graminearum cultivées sur milieu PDB à 25°C et à l'obscurité pendant 24 et 72 h d'incubation.**

| **Temps d'incubation** | **Efficacité fongicide (% du témoin non traité) ^{a}** | | | | | | | | |
|---|---|---|---|---|---|---|---|---|---|
| | **0.010 mg/ml** | **0.025 mg/ml** | **0.050 mg/ml** | **0.125 mg/ml** | **0.250 mg/ml** | **0.375 mg/ml** | **0.5 mg/ml** | **1.25 mg/ml** | **5.0 mg/ml** |
| **24 h** | **2.0** | **5.9** | **14.3** | **56.2** | **100.0** | **100.0** | **100.0** | **100.0** | **100.0** |
| **72 h** | **0.3** | **0.3** | **0.2** | **1.1** | **100.0** | **100.0** | **100.0** | **100.0** | **100.0** |

| | | | | | | | | | |
|---|---|---|---|---|---|---|---|---|---|
| ^{a}L'efficacité fongicide de l'extrait de micro-algue a été déterminée après 24 et 72 h d'incubation sur milieu PDB à 25°C et à l'obscurité par mesure de la densité optique à 590 nm dans chaque puit de la microplaque à 96 puits. Chaque valeur correspond à la moyenne de 3 répétitions par condition testée | | | | | | | | | |

**Tableau 3. Efficacité de l'extrait de micro-algue in vitro à l'encontre de Zymoseptoria tritici souche Mg StA, Rhizoctonia solani souche Rsol AG3, Microdochium majus souche Mmaj E11 et Fusarium graminearum souche Fg 1.**

| **Agent Pathogène** | **Efficacité fongicide (% du témoin non traité) ^{a}** | | | | | | |
|---|---|---|---|---|---|---|---|
| | **0.050 mg/ml** | **0.125 mg/ml** | **0.250 mg/ml** | **0.5 mg/ml** | **1.25 mg/ml** | **2.5 mg/ml** | **5.0 mg/ml** |
| ***Z. tritici*** | 5.0 | 98.4 | 100.0 | 100.0 | 100.0 | 100.0 | 100.0 |
| ***R. solani*** | 5.7 | 71.1 | 100.0 | 100.0 | 100.0 | 100.0 | 100.0 |
| ***M. majus*** | 16.2 | 42.4 | 100.0 | 100.0 | 100.0 | 100.0 | 100.0 |
| ***F. graminearum*** | 15.8 | 31.7 | 98.9 | 100.0 | 100.0 | 100.0 | 100.0 |

| | | | | | | | |
|---|---|---|---|---|---|---|---|
| ^{a}L'efficacité fongicide de l'extrait de micro-algue a été déterminée après 72 h d'incubation pour *M. majus* et *F. graminearum* ou 5 jours d'incubation pour *Z. tritici* et *R. solani* sur milieu PDB à 25°C et à l'obscurité par mesure de la densité optique à 590 nm dans chaque puit de la microplaque à 96 puits. Chaque valeur correspond à la moyenne de 3 répétitions par condition testée. | | | | | | | |

**Tableau 4. DE₅₀ et CMI (mg m.a./ml) de l'extrait de micro-algue à l'encontre de Zymoseptoria tritici souche Mg StA, Rhizoctonia solani souche Rsol AG3, Microdochium majus souche Mmaj E11 et Fusarium graminearum souche Fg 1.**

| **Agent Pathogène** | **Efficacité fongicide (mg m.a./ml) ^{a}** | |
|---|---|---|
| | **DE₅₀** | **CMI** |
| ***Z. tritici*** | 0,084 +/- 0,010 | 0,126 +/- 0,006 |
| ***R. solani*** | 0,106 +/- 0,015 | 0,189 +/- 0,041 |
| ***M. majus*** | 0,123 +/- 0,009 | 0,266 +/- 0,019 |
| ***F. graminearum*** | 0,136 +/- 0,006 | 0,290 +/- 0,003 |

| | | |
|---|---|---|
| ^{a}DE50: Dose efficace d'extrait de micro-algue réduisant de 50% le développement des agents pathogènes testés et CMI: Concentration Minimale Inhibitrice, plus petite concentration qui inhibe de 100% le développement des agents pathogènes testés. Chaque valeur correspond à la moyenne de 3 répétitions par condition testée +/- écart type. | | |

**Tableau 5. Efficacité de l'extrait de micro-algue in vitro à l'encontre de Phytophthora infestans souche PiF14, Fusarium oxysporum f. sp. cubense souche CBS 102013.**

| **Agent Pathogène** | **Efficacité fongicide (% du témoin non traité) ^{a}** | | | | | | |
|---|---|---|---|---|---|---|---|
| | **0.050 mg/ml** | **0.125 mg/ml** | **0.250 mg/ml** | **0.5 mg/ml** | **1.25 mg/ml** | **2.5 mg/ml** | **5.0 mg/ml** |
| ***P. infestans*** | 2,1 | 4,4 | 8,7 | 35,9 | 75,8 | 98,7 | 100.0 |
| ***F. oxysporum* f. sp. *cubense*** | 0,0 | 14,0 | 100.0 | 100.0 | 100.0 | 100.0 | 100.0 |

| | | | | | | | |
|---|---|---|---|---|---|---|---|
| ^{a}L'efficacité fongicide de l'extrait de micro-algue a été déterminée après 72 h d'incubation pour les 3 agents pathogènes à 20°C et à l'obscurité par mesure de la densité optique à 590 nm dans chaque puit de la microplaque à 96 puits. Chaque valeur correspond à la moyenne de 3 répétitions par condition testée. | | | | | | | |

**Tableau 6. DE₅₀ et CMI (mg m.a./ml) de l'extrait de micro-algue à l'encontre de Phytophthora infestans souche PiF14, Fusarium oxysporum f. sp. cubense souche CBS 102013**

| **Agent Pathogène** | **Efficacité fongicide (mg m.a./ml) ^{a}** | |
|---|---|---|
| | **DE₅₀** | **CMI** |
| ***P. infestans*** | 0,70 +/- 0,040 | 5,0 +/- 0,0 |
| ***F. oxysporum* f. sp. *cubense*** | 0,175 +/- 0,02 | 0,25 +/- 0,03 |

| | | |
|---|---|---|
| ^{a}DE₅₀: Dose efficace d'extrait de micro-algue réduisant de 50% le développement des agents pathogènes testés et CMI: Concentration Minimale Inhibitrice, plus petite concentration qui inhibe de 100% le développement des agents pathogènes testés. Chaque valeur correspond à la moyenne de 3 répétitions par condition testée +/- écart type. ^{B}a.i.: Aucune inhibition, même à la plus forte concentration testée de 5 mg/ml. | | |

### Exemple 9 : Test in vitro et in planta (plantules de pommier) vis-à-vis de la tavelure du pommier (Venturia inaequalis)

L'activité antifongique de l'extrait D à 5 g/L a été testé sur boite de petri en présence de spores de *Venturia inaequalis* mises 2h après ou 3h avant l'extrait D. Après 48h d'incubation, le pourcentage de germination des spores a été évalué. La présence de l'extrait D, 2h avant ou 3h après l'inoculation, inhibe complètement la germination des spores de *V. inaequalis* contrairement à la présence de l'eau.

Les tests *in planta* sur plantules de pommier ont été réalisés selon le protocole suivant :
2 modalités testées : pulvérisation de l'extrait D à 5g/L 2h avant inoculation (Extraitavant) et pulvérisation de l'extrait D à 5g/L 3h après inoculation (Extraitaprès)
3 modalités témoin :
   ∘2 modalités témoin non traitées (pulvérisation d'eau aux mêmes dates que le produit) (EauAvant et EauAprès)
   ∘ 1 modalité avec un fongicide de référence (captane)

Inoculation artificielle des plantes avec une souche de *Venturia inaequalis à* 100 000 spores/ml. Notations des symptômes de tavelure à 4 dates après l'inoculation (estimation visuelle du % de surface tavelée)

Matériel biologique :
- Plantules issues de semis de pommier en motte-fertis et de 3 semaines environ - à partir de pépins prélevés sur la variété Gala (pommes récoltées dans un verger planté avec Gala et Elstar)
- Spores de *Venturia inaequalis* sur cellophane-Souche EUB04

3 boites de 12 plantules pour chaque modalité
J+9, J+14, J+17, J+21 : Notation en pourcentage de surface foliaire atteinte (de 0 à 100% avec une échelle de 10% en 10%)

Les conditions de normalité et d'homogénéité des variances des résidus n'étant pas respectées, un test non paramétrique de Kruskall-Wallis a été réalisé. Ce test étant significatif, les médianes des différentes modalités ont été comparées paire par paire grâce au test de Nemenyi basé sur la distance de Tukey. Ces analyses ont été réalisées avec le logiciel R (version 3.1.2).

L'application de l'extrait de l'Exemple 2 n'a pas entraîné de phytotoxicité sur les plantes : aucune réaction de nécrose, chlorose ou boursoufflure n'a été observée.

Observation à J9 : aucun symptôme de tavelure sur les 5 modalités.

Sur les témoins non traités (EauAvant et EauAprès), la tavelure s'est bien développée pour atteindre une médiane de 50 à 60% à J21. Pas de différence significative entre les deux modalités eau quelle que soit la date.

Sur le témoin traité au captane, aucun développement de tavelure n'a été observé.

Sur les modalités traitées (Extraitavant et Extraitaprès), le développement de la tavelure est significativement plus faible que pour les témoins non traités quelle que soit la date d'observation. Quelle que soit la date d'observation, il n'y a pas de différence significative entre la modalité Extraitavant et la modalité captane. 14 jours après inoculation, il n'y a pas non plus de différence significative entre la modalité ExtraitAprès et le captane, mais à 17 et 21 jours après inoculation, il y a significativement plus de tavelure pour la modalité ExtraitAprès que pour la modalité Captane. Il n'y a pas de différences significatives entre ExtraitAvant et Extraitaprès.

Très bonne efficacité de l'extrait d'*A*. *carterae* vis-à-vis de la tavelure lorsqu'il est appliqué 2h avant inoculation ou 3h après inoculation.

### Exemple 10 Test in planta sur différentes espèces de Colletotrichum

Afin de déterminer si l'extrait d'A. carterae a un effet biocide sur l'ascomycète *Colletotrichum,* deux espèces différentes de *Colletotrichum* ont été testées : *Colletotrichum fructicola* et *Colletotrichum orbiculare.* Pour ces deux espèces, le mode opératoire est identique : un volume d'extrait à une concentration donnée (de 1 à 10 g/L), ou un volume d'eau, a été mélangé avec un volume contenant 2.10⁶ spores de *Colletotrichum fructicola* ou 2.10⁶ spores de *Colletotrichum orbiculare,* puis des spots de 10 µl ont été appliqués sur feuilles détachées de fraisier ou de concombre, respectivement. Les feuilles ont été gardées dans une boîte de petri sous une atmosphère humide pendant 6 jours à 24°C dans une chambre de culture (jour/nuit ; 14H/12H). Les résultats montrent que l'extrait d'*A*. *carterae* inhibe totalement la croissance de ces deux espèces de *Colletotrichum à* partir d'une concentration de 1g/L.

### Exemple 11 : Test de l'intégrité de la paroi et la membrane plasmique des conidiospores de Fusarium graminearum

Les conidiospores de *F. graminearum* sont incubés 1h en présence de l'extrait selon l'exemple 2 (5 g/L) ou du même extrait (5 g/L) inactivé par une température supérieure à 60°C. L'iodure de propidium est rajouté afin de déterminer l'intégrité membranaire des spores.

Les photographies montrent une coloration des conidiospores avec l'iodure de propidium en présence de l'extrait selon l'exemple 2 (5 g/L) et pas en présence du même extrait (5 g/L) inactivé.

Ces résultats montrent que l'extrait selon l'exemple 2 agit en augmentant de façon significative la porosité de la paroi et de la membrane plasmique des conidiospores de *Fusarium graminearum.*

L'ensemble de ces résultats démontrent que l'extrait issu de *A. carterae* possède une activité antifongique notable, que cela soit dans des conditions *in vitro* ou *in planta,* sur un spectre large de champignons phytopathogènes.

### REFERENCES BIBLIOGRAPHIQUES

(1) Arseniuk, E., Foremska, E., Goral, T., Chelkowski, J. 1999. Fusarium head blight reactions and accumulation ofdeoxynivalenol (DON) and some of its derivatives in kernels of wheat, triticale and rye. Journal of Phytopathology 147, 577-590
(2) Devi P, Wahidulla S, Kamat T and D'Souza L (2011). Screening marine organisms for antimicrobial activity against clinical pathogens. Indian J.Geomar.Sci. (40) 338-346.
(3) Mayer AM, Rodriguez AD, Taglialatela-Scafati O, Fusetani N (2013). Marine pharmacology in 2009-2011: marine compounds with antibacterial, antidiabetic, antifungal, anti-inflammatory, antiprotozoal, antituberculosis, and antiviral activities; affecting the immune and nervous systems, and other miscellaneous mechanisms of action. Mar Drugs. 11(7):2510-73
(4) Bowler, C., Vardi, A., & Allen, A. E. (2010). Oceanographic and biogeochemical insights from diatom genomes. Annual Review of Marine Science, 2, 333-65. doi:10.1146/annurev-marine-120308-081051
(5) Murray S, Garby T, Hoppenrath M, Neilan BA (2012). Genetic diversity, morphological uniformity and polyketide production in dinoflagellates (Amphidinium, Dinoflagellata). PLoS One. 7(6)
(6) Morsy N, Houdai T, Matsuoka S, Matsumori N, Adachi S, et al. (2006). Structures of new amphidinols with truncated polyhydroxyl chain and their membrane-permeabilizing activities. Bioorganic and Medicinal Chemistry 14: 6548-6554.
(7) Nuzzo G, Cutignano A, Sardo A, Fontana A (2014). Antifungal Amphidinol 18 and its 7-sulfate derivative from marine dinoflagellate Amphidinium carterae, J. Nat. Prod., , 1524-1527.

## Revendications

1. Procédé de lutte contre les champignons, et/ou les oomycètes des plantes et semences de culture **caractérisé par** les étapes suivantes :
- Récolte de cellules fraiches d'une ou plusieurs micro-algue(s) du genre *Amphidinium ;*
- Congélation et/ou lyophilisation desdites cellules ;
- Suspension dudit lyophilisat ou desdites cellules congelées dans de l'eau dans un rapport en poids de 1:200 à 1:2 à une température supérieure à 60°C ;
- Application sur des plantes de culture et/ou enrobage de semences de l'extrait ainsi obtenu.

2. Procédé selon la revendication 1 dans laquelle la ou l'une des micro-algues du genre Amphidinium est Amphidinium carterae.

3. Procédé selon la revendication 1 ou 2, ledit extrait comprenant l'amphidinol 18 ou l'amphidinol 19, de préférence l'amphidinol 18.

4. Procédé selon l'une quelconque des revendications 1 à 3, dans lequel les cellules fraiches sont récoltées à une concentration cellulaire comprise entre 5.10⁴ cellules/ml et 5.10⁶ cellules/ml.

5. Procédé selon l'une quelconque des revendications 1 à 4, dans lequel lesdits champignons pathogènes des plantes et semences de culture sont des champignons pathogènes des plantes et semences de culture des genres *Fusarium, Colletotrichum, Mycosphaerella, Phytophthora* et *Alternaria,* de préférence les couples champignons vs. plantes de culture *Triticum sativum*/*Mycosphaerella graminicola - Triticum sativum*/*Fusarium graminearum - Solanum tuberosum*/*Phytophtora infestans - Vinis vitifera*/*Plasmospora viticola - Vinis vitiferalerysiphe necator - Lycopersicon esculentum*/*Phytophtora infestans.*

6. Procédé selon la revendication 5 dans lequel lesdits champignons pathogènes des plantes et semences de culture des genres *Fusarium, Colletotrichum, Mycosphaerella, Phytophthora* et *Alternaria* sont choisis dans le groupe constitué de *Fusarium oxysporum,* Fusarium solani, Fusarium avenaceum, Fusarium culmorum, *Fusarium graminearum, Fusarium moniliforme, Fusarium poae, Fusarium proliferatum, Fusarium sporotrichioides, Fusarium subglutinans, Fusarium tricinctum, Colletotrichum acutatum, Colletotrichum graminicola, Colletotrichum coffeanum, Colletotrichum gloeosporioides, Mycosphaerella graminicola, Phytophthora infestans, Alternaria solani* et *Alternaria brassisicola.*

## Patentansprüche

1. Verfahren zur Bekämpfung von Pilzen und/oder von Oomyceten von Kulturpflanzen und -saatgut, **gekennzeichnet durch** die folgenden Schritte:
- Ernten frischer Zellen einer oder mehrerer Mikroalge(n) der Gattung *Amphidinium;*
- Einfrieren und/oder Gefriertrocknen der Zellen;
- Suspendieren des Lyophilisats oder der gefrorenen Zellen in Wasser in einem Gewichtsverhältnis von 1:200 bis 1:2 bei einer Temperatur über 60 °C;
- Auftragen auf die Kulturpflanzen und/oder Beschichten des Saatguts mit dem derart erhaltenen Extrakt.

2. Verfahren nach Anspruch 1, wobei die oder eine der Mikroalgen der Gattung Amphidinium Amphidinium carterae ist.

3. Verfahren nach Anspruch 1 oder 2, wobei der Extrakt Amphidinol 18 oder Amphidinol 19, vorzugsweise Amphidinol 18, umfasst.

4. Verfahren nach einem der Ansprüche 1 bis 3, wobei die frischen Zellen in einer Zellkonzentration zwischen 5.10⁴ Zellen/ml und 5.10⁶ Zellen/ml geerntet werden.

5. Verfahren nach einem der Ansprüche 1 bis 4, wobei die für Kulturpflanzen und -saatgut pathogenen Pilze pathogene Pilze für Kulturpflanzen und -saatgut der Gattungen *Fusarium, Colletotrichum, Mycosphaerella, Phytophthora* und *Alternaria* sind, vorzugsweise die Pilz-Kulturpflanzen-Paare *Triticum sativum*/*Mycosphaerella graminicola - Triticum sativum*/*Fusarium graminearum - Solanum tuberosum*/*Phytophtora infestans* - *Vinis vitifera*/*Plasmospora viticola - Vinis vitifera*/*Erysiphe necator - Lycopersicon esculentum*/*Phytophtora infestans.*

6. Verfahren nach Anspruch 5, wobei die für Kulturpflanzen und -saatgut pathogenen Pilze der Gattungen *Fusarium, Colletotrichum, Mycosphaerella, Phytophthora* und *Alternaria* aus der Gruppe ausgewählt sind, die aus *Fusarium oxysporum,* Fusarium solani, Fusarium avenaceum, Fusarium culmorum, *Fusarium graminearum, Fusarium moniliforme, Fusarium poae, Fusarium proliferatum, Fusarium sporotrichioides, Fusarium subglutinans, Fusarium tricinctum, Colletotrichum acutatum, Colletotrichum graminicola, Colletotrichum coffeanum, Colletotrichum gloeosporioides, Mycosphaerella graminicola, Phytophthora infestans, Alternaria solani* und *Alternaria brassisicola* besteht.

## Claims

1. A process for controlling crop plant and seed pathogenic fungi and/or oomycetes, **characterized by** the following steps:
- Harvesting fresh cells from one or more microalga(e) of the genus *Amphidinium;*
- Freezing and/or freeze-drying said cells;
- Suspending said lyophilisate or said frozen cells in water in a weight ratio of 1:200 to 1:2 at a temperature greater than 60°C;
- Applying the extract thus obtained to crop plants and/or coating seeds with said extract.

2. Process according to claim 1 wherein the or one of the microalgae of the *genus Amphidinium* is *Amphidinium carterae.*

3. Process according to claim 1 or 2, said extract comprising amphidinol 18 or amphidinol 19, preferably amphidinol 18.

4. Process according to any one of claims 1 to 3, wherein the fresh cells are harvested at a cell concentration comprised between 5.10⁴ cells/ml and 5.10⁶ cells/ml.

5. Process according to any one of claims 1 to 4, wherein said crop plant and seed pathogenic fungi are fungi pathogenic to crop plants and seeds of the genera *Fusarium, Colletotrichum, Mycosphaerella, Phytophthora* and *Alternaria,* preferably the pairs of fungi vs. crop plants *Triticum sativum*/*Mycosphaerella graminicola - Triticum sativum*/*Fusarium graminearum - Solanum tuberosum*/*Phytophtora infestans - Vinis vitifera*/*Plasmospora viticola - Vinis vitifera*/*Erysiphe necator - Lycopersicon esculentum*/*Phytophtora infestans.*

6. Process according to claim 5 wherein said fungi pathogenic to crop plants and seeds of the genera *Fusarium, Colletotrichum, Mycosphaerella, Phytophthora* and *Alternaria* are selected from the group consisting of *Fusarium oxysporum, Fusarium solani, Fusarium avenaceum, Fusarium culmorum, Fusarium graminearum, Fusarium moniliforme, Fusarium poae, Fusarium proliferatum, Fusarium sporotrichioides, Fusarium subglutinans, Fusarium tricinctum, Colletotrichum acutatum, Colletotrichum graminicola, Colletotrichum coffeanum, Colletotrichum gloeosporioides, Mycosphaerella graminicola, Phytophthora infestans, Alternaria solani* and *Alternaria brassisicola.*
